(19) Europäisches Patentamt
European Patent Office
Office européen des brevets

(11) **EP 1 487 575 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**01.04.2009 Patentblatt 2009/14**

(45) Hinweis auf die Patenterteilung:
**14.12.2005 Patentblatt 2005/50**

(21) Anmeldenummer: **03708212.0**

(22) Anmeldetag: **12.03.2003**

(51) Int Cl.:
***B01J 27/198*** (2006.01)

(86) Internationale Anmeldenummer:
**PCT/EP2003/002502**

(87) Internationale Veröffentlichungsnummer:
**WO 2003/078310 (25.09.2003 Gazette 2003/39)**

(54) **VERFAHREN ZUR HERSTELLUNG EINES VANADIUM, PHOSPHOR UND SAUERSTOFF ENTHALTENDEN KATALYSATORS**

METHOD FOR THE PRODUCTION OF A CATALYST CONTAINING VANADIUM, PHOSPHORUS, AND OXYGEN

PROCEDE DE PRODUCTION D'UN CATALYSEUR CONTENANT DU VANADIUM, DU PHOSPHORE ET DE L'OXYGENE

(84) Benannte Vertragsstaaten:
**AT BE BG CH CY CZ DE DK EE ES FI FR GB GR HU IE IT LI LU MC NL PT RO SE SI SK TR**

(30) Priorität: **15.03.2002 DE 10211446**

(43) Veröffentlichungstag der Anmeldung:
**22.12.2004 Patentblatt 2004/52**

(73) Patentinhaber: **BASF SE**
**67056 Ludwigshafen (DE)**

(72) Erfinder:
 • **WEIGUNY, Jens**
 **Shanghai 200336m (CN)**
 • **STORCK, Sebastian**
 **68167 Mannheim (DE)**
 • **DUDA, Mark**
 **67071 Ludwigshafen (DE)**
 • **DOBNER, Cornelia**
 **67227 Frankenthal (DE)**
 • **FELDER, Raimund**
 **67434 Neustadt (DE)**

(74) Vertreter: **Isenbruck, Günter**
**Isenbruck Bösl Hörschler Wichmann Huhn LLP**
**Patentanwälte**
**Theodor-Heuss-Anlage 12**
**68165 Mannheim (DE)**

(56) Entgegenhaltungen:
EP-A- 0 275 671    WO-A-01/36364
WO-A-02/024620    DE-A- 10 046 072
US- - 4 699 985    US-A- 4 534 780
US-A- 4 567 158    US-A- 4 699 985
US-A- 4 719 332    US-A- 5 137 860
US-A- 5 168 090    US-A- 5 275 996

• DATABASE WPI Section Ch, Week 199623 Derwent Publications Ltd., London, GB; Class J09, AN 1996-225929 XP002244790 & JP 08 086571 A (KAO CORP), 2. April 1996 (1996-04-02)
• Kirk-Othme, Encyclopedia of Chemical Technology, 3 Auflage, Band 11 , Seiten 551-557
• BEGAKIS: 'Short communication, An Inexpensive Thick Film Furnace' ELECTROCOMPONENT SCIENCE AND TECHNOLOGY Bd. 3, 1976, Seiten 113 - 115
• MPR, Dezember 1996, Seite 42, " Belt Furnaces"
• KAZMIEROWICZ C. ET AL: 'MONITORING BELT FURNACE TEMPERATURES: A NEW WAY' HYBRID CIRCUIT TECHNOLOGY November 1988, Seiten 39 - 45
• M.P.M.A Limpens,PhD Thesis, 01.11.2000, " A Design Tool for Conveyor Belt Furnaces, Seiten 1-90
• BRIEM K.: 'Wärmebehandlung von Massengutteilen....' GASWÄRMWE INTERNATIONAL Bd. 47, Nr. 9, September 1998, Seiten 439 - 444
• A.Enderlein et al., " A new Conveyor Furnace for Thick Film Firing...", International Microelectronic Symposium , Chicago, 11-13.10.1971, Seiten 4-3-1 bis 4-3-4

**EP 1 487 575 B2**

**Beschreibung**

Die vorliegende Erfindung betrifft ein Verfahren zur Herstellung eines für die heterogenkatalytische Gasphasenoxidation eines Kohlenwasserstoffs mit mindestens vier Kohlenstoffatomen zu Maleinsäureanhydrid geeigneten, Vanadium, Phosphor und Sauerstoff enthaltenden Katalysators, bei dem man einen entsprechenden Vanadium, Phosphor und Sauerstoff enthaltenden Katalysatorprecursor, welcher Partikel mit einem gemittelten Durchmesser von mindestens 2 mm aufweist, durch Kalzinierung in eine katalytisch aktive Form überführt.

Maleinsäureanhydrid ist ein wichtiges Zwischenprodukt bei der Synthese von γ-Butyrolacton, Tetrahydrofuran und 1,4-Butandiol, welche ihrerseits als Lösungsmittel eingesetzt werden oder beispielsweise zu Polymeren, wie Polytetrahydrofuran oder Polyvinylpyrrolidon weiterverarbeitet werden.

Die Herstellung von Maleinsäureanhydrid durch Oxidation von Kohlenwasserstoffen wie n-Butan, n-Butenen oder Benzol an geeigneten Katalysatoren ist schon seit langem bekannt. Im Allgemeinen werden hierzu Vanadium-, Phosphor- und Sauerstoff enthaltende Katalysatoren (sogenannte VPO-Katalysatoren) eingesetzt (siehe Ullmann's Encyclopedia of Industrial Chemistry, 6th edition, 2000 electronic release, Chapter "MALEIC AND FUMARIC ACIDS, Maleic Anhydride - Production").

Da die Oxidation der genannten Kohlenwasserstoffe zu Maleinsäureanhydrid stark exotherm ist, wird die Umsetzung in der Regel in einem salzbad-gekühlten Festbett-Rohrbündelreaktor durchgeführt. Je nach Größe der Anlage weist dieser wenige tausend bis mehrere zehntausend, mit Katalysator gefüllte Rohre auf. Die gebildete Reaktionswärme wird über die Wandung der Katalysator-gefüllten Rohre an das umgebende Salzbad, in der Regel eine eutektische Mischung von Kalium- und Natriumnitrat, übertragen und abgeführt. Trotz dieser Salzbad-Kühlung stellt sich über die Länge und den Querschnitt der Katalysator-gefüllten Rohre keine einheitliche Temperatur ein. Es kommt zur Ausbildung überhitzter Bereiche, sogenannter Heißpunkte ("Hot Spots"). So ist die Kohlenwasserstoff-Konzentration des Reaktionsgemisches in der Nähe der Eintrittsstelle der Katalysator-gefüllten Rohre am höchsten und in der Nähe der Austrittsstelle am niedrigsten, was zur Bildung der genannten überhitzten Bereiche in der Nähe der Eintrittsstelle führt.

Die Bildung überhitzter Bereiche in den Katalysator-gefüllten Rohren ist aus verschiedenen Gründen nachteilig. In den überhitzten Bereichen kommt es zu einer übermäßigen Oxidation über die gewünschte Oxidationsstufe hinaus. Ferner beeinflusst die übermäßige thermische Belastung die Katalysatorleistung und -lebensdauer. Da mit steigender Temperatur auch die Reaktionsgeschwindigkeit steigt und somit noch mehr Wärme produziert wird, kann die Bildung überhitzter Bereiche schließlich zu einem unkontrollierten Reaktionsverlauf führen, der ein explosionsartiges Durchgehen der Reaktion zur Folge haben kann.

Die Einstellung einer niedrigen Reaktionstemperatur würde zwar den obengenannten Nachteilen der überhitzten Bereiche entgegenwirken, jedoch zu einem unwirtschaftlich geringen Umsatz und somit zu einer unwirtschaftlich geringen Ausbeute an Wertprodukt führen.

Aus wirtschaftlichen und sicherheitstechnischen Gründen wird der Rohrbündelreaktor daher derart betreiben, dass die Reaktionstemperatur zur Sicherstellung einer wirtschaftlich attraktiven Ausbeute so hoch wie möglich ist, jedoch die in den einzelnen Rohren sich einstellenden überhitzten Bereiche nicht zu einem unkontrollierten Reaktionsverlauf führen. In der Praxis sind die sich in den einzelnen Rohren einstellenden Temperaturen nicht exakt gleich. Entscheidend für den Betrieb des gesamten Rohrbündelreaktors ist letztendlich die Reaktionstemperatur des heißesten Rohres. Je stärker die Streuung der sich einstellenden Temperaturen ist, desto niedriger ist die mittlere Reaktionstemperatur aus sicherheitstechnischen Gründen einzustellen. Eine geringe Streuung ist daher wünschenswert. Beim Befüllen der Rohre mit Katalysator wird daher genau darauf geachtet, dass in allen Rohren dieselbe Katalysatormenge beziehungsweise auch dieselbe Katalysatormischung vorliegt. Es ist ferner üblich, nach dem Befüllen der Rohre mit dem Katalysator einen sogenannten Druckabgleich durchzuführen und durch Hinzufügen von weiterem Inertmaterial in allen Rohren den gleichen Strömungswiderstand einzustellen. Diese Maßnahme stellt sicher, dass beim Betrieb alle Rohre mit derselben Gasmenge durchströmt werden.

Erfindungsgemäß wurde erkannt, dass auch durch die oben beschriebenen Maßnahmen teilweise noch erhebliche Unterschiede in den sich einstellenden Temperaturen der einzelnen Katalysator-befüllten Rohre bestehen. Es wurde ferner erfindungsgemäß erkannt, dass diese Unterschiede zum Großteil auf den bei der Befüllung der Rohre eingesetzten Katalysators zurückzuführen sind. Weist nämlich dieser in seiner Gesamtheit Partikel unterschiedlicher Aktivität auf, so wird ein Teil des Katalysators zwangsläufig bei einer Temperatur betrieben, die mehr oder weniger weit vom optimalen Temperaturbereich der jeweiligen Katalysatorpartikel abweicht. Dies ist mit Einbußen bei der Ausbeute und der Katalysatorlebensdauer verbunden.

Die im Allgemeinen eingesetzten Vanadium-, Phosphor- und Sauerstoff enthaltenden Katalysatoren werden in der Regel wie folgt hergestellt:

(1) Synthese eines Vanadylphosphat-Hemihydrat-Precursors ($VOHPO_4 \cdot \frac{1}{2}H_2O$) aus einer fünfwertigen Vanadium-Verbindung (z.B. $V_2O_5$), einer fünf- oder dreiwertigen Phosphor-Verbindung (z.B. Ortho-, Pyro- und/oder Polyphosphorsäure, Phosphorsäureester oder phosphorige Säure) und einem reduzierend wirkenden Alkohol (z.B. Isobutanol), Isolierung des Niederschlags und Trocknung, gegebenenfalls Formgebung (z.B. Tablettierung); und

(2) Präformierung zum Vanadylpyrophosphat ($(VO)_2P_2O_7$) durch Kalzinierung.

Varianten und verschiedene Ausführungsformen der Katalysatorherstellung sind beispielsweise in US 4,567,158, US 4,996,179, US 5,137,860, US 5,506,187, US 5,530,144 und US 5,773,382 beschrieben.

US 4,567,158 offenbart die Kalzinierung der geformten Vanadylphosphat-Hemihydrat-Precursor-Tabletten in einem fremdbelüfteten Ofen, wobei eine ein- und eine zweistufige Kalzinierung beschrieben sind. Bei der einstufigen Kalzinierung werden die Precursor-Tabletten unter Luft bei 350°C behandelt. Bei der zweistufigen Kalzinierung werden die Precursor-Tabletten zunächst unter Luft bei 350 bis 400°C und anschließend unter einer Stickstoff/Wasserdampf-Atmosphäre bei 330 bis 500°C kalziniert.

US 5,137,860 lehrt die Kalzinierung der geformten Vanadylphosphat-Hemihydrat-Precursor-Tabletten auf mit Löchern versehenen Edelstahl-Blechen in einem Hordenofen. Hierzu werden die Precursor-Tabletten zunächst in einer Sauerstoff-, Wasserdampf- und gegebenenfalls Inertgas-haltigen Atmosphäre bei einer Temperatur bis 300°C behandelt, dann einer Temperaturerhöhung auf größer 350°C und kleiner 550°C zur Einstellung der Vanadium-Oxidationsstufe unterzogen und anschließend die Temperaturbehandlung unter einer nichtoxidierenden, Wasserdampf-haltigen Atmosphäre mit einem Wassergehalt von 25 bis 75 mol-% fortgesetzt.

In US 5,773,382 ist der Einsatz von Porenbildnern in der Herstellung von Vanadium, Phosphor und Sauerstoff enthaltenden Katalysatoren beschrieben. Die den Porenbildner enthaltenden Vanadylphosphat-Hemihydrat-Precursor-Tabletten werden dabei zunächst auf Blechen in einem Hordenofen unter Luftzufuhr bei einer Temperatur von 165 bis 255°C von dem Porenbildner befreit und anschließend in einem weiteren Hordenofen wie in US 5,137,860 beschrieben kalziniert.

US 4,996,179 lehrt die Präformierung eines durch Fällung, Eindampfung und Trocknung erhaltenen Vanadium, Phosphor und Sauerstoff enthaltenden Katalysatorprecursorpulvers in Luft oder einem Stickstoff-Luft-Gemisch bei 300 bis 370°C in einem Drehrohr- oder Wirbelschichtofen. Das erhaltene präformierte Katalysatorprecursorpulver wird anschließend tablettiert und in einer inerten Atmosphäre bei einer Temperatur von 343 bis 704°C sowie anschließend in einer Sauerstoff enthaltenden Atmosphäre bei erhöhter Temperatur kalziniert.

In US 5,506,187 ist die Kalzinierung eines durch Fällung, Eindampfung und Trocknung erhaltenen Vanadium, Phosphor und Sauerstoff enthaltenden Katalysatorprecursorpulvers in Luft beziehungsweise Luft/Wasserdampf bei 288 bis 427°C in einem Drehrohrofen beschrieben.

US 5,530,144 lehrt die Kalzinierung von Vanadylphosphat-Hemihydrat-Precursorn unter Stickstoff, Luft oder deren Mischungen bei einer Temperatur von 350 bis 700°C in einem geeigneten Kalzinierapparat. Als allgemeine Beispiele sind Wirbelschichtofen, Drehrohrofen und kontinuierlich betriebener Muffelofen genannt.

Es bestand die Aufgabe, ein Verfahren zur Herstellung eines partikulären Vanadium, Phosphor und Sauerstoff enthaltenden Katalysators für die heterogenkatalytische Gasphasenoxidation eines Kohlenwasserstoffs mit mindestens vier Kohlenstoffatomen zu Maleinsäureanhydrid bereit zu stellen, welches auch bei der Produktion größerer Mengen im Tonnen-Maßstab zu einem Katalysator führt, welcher, eingesetzt in einem Rohrbündelreaktor, zu einer sehr geringen Streuung der sich in den einzelnen Rohren einstellenden Temperaturen führt und somit eine weitergehende Optimierung hinsichtlich eines hohen Umsatzes, einer hohen Selektivität, einer hohen Ausbeute und einer langen Katalysatorlebensdauer bei gleichzeitig hoher Sicherheit in Bezug auf das Durchgehen der Reaktion ermöglicht.

Demgemäß wurde ein Verfahren zur Herstellung eines für die heterogenkatalytische Gasphasenoxidation eines Kohlenwasserstoffs mit mindestens vier Kohlenstoffatomen zu Maleinsäureanhydrid geeigneten, Vanadium, Phosphor und Sauerstoff enthaltenden Katalysators, bei dem man einen entsprechenden Vanadium, Phosphor und Sauerstoff enthaltenden Katalysatorprecursor, welcher Partikel mit einem gemittelten Durchmesser von mindestens 2 mm aufweist, durch Kalzinierung in eine katalytisch aktive Form überführt, gefunden, das daduch gekennzeichnet ist, dass man einen Strom des Katalysatorprecursors zur Kalzinierung mit im Wesentlichen konstanter Geschwindigkeit auf einem Förderband durch mindestens eine Kalzinierungszone n der Länge $l_n$ führt, wobei die zeitliche Schwankung der Gastemperatur vom Sollwert an jeder Stelle im Bereich des Stromes des Katalysatorprecursors nach der halben Länge der Kalzinierungszone $l_n/2$ jeweils $\leq 5°C$ und die lokale Differenz der Gastemperatur zwischen beliebigen Stellen im Bereich des Stromes des Katalysatorprecursors nach der halben Länge der Kalzinierungszone $l_n/2$ jeweils $\leq 5°C$ betragen, **dadurch gekennzeichnet, dass** man den Katalysatorprecursor

(a) in mindestens einer Kalzinierungszone in einer oxidierenden Atmosphäre mit einem Sauerstoff-Gehalt von 2 bis 21 Vol.-% auf eine Temperatur von 200 bis 350°C aufheizt und unter diesen Bedingungen bis zur Einstellung der gewünschten mittleren Oxidationsstufe des Vanadiums beläßt; und

(b) in mindestens einer weiteren Kalzinierungszone in einer nicht-oxidierenden Atmosphäre mit einem Sauerstoff-Gehalt von $\leq 0,5$ Vol.-% und einem Wasserstoffoxid-Gehalt von 20 bis 75 Vol.-% auf eine Temperatur von 300 bis 50.0°C aufheizt und ≥ 0,5 Stunden unter diesen Bedingungen beläßt.

Wesentlich beim erfindungsgemäßen Verfahren ist eine möglichst gleichmäßige Kalzinierung des Vanadium, Phosphor und Sauerstoff enthaltenden Katalysatorprecursors, insbesondere in Bezug auf die umgebende Gaszusammensetzung, auf die umgebende Temperatur und die jeweilige Behandlungszeit unter diesen Bedingungen. Sie wird erfindungsgemäß dadurch sichergestellt, dass man

- den partikulären Katalysatorprecursor mit im Wesentlichen konstanter Geschwindigkeit durch mindestens eine Kalzinierungszone führt; und

- innerhalb der Kalzinierungszone im Bereich des Stromes des Katalysatorprecursors für eine möglichst hohe räumliche und zeitliche Temperaturkonstanz sorgt.

Unter dem Begriff Kalzinierung sind allgemein thermische Behandlungsschritte zu verstehen, einschließlich einer Verdampfung oder Zersetzung von im Katalysatorprecursor enthaltenden Komponenten, wie etwa die Trocknung oder die Zersetzung eines Hilfsstoffes.

Im Allgemeinen setzt man zur Durchführung des erfindungsgemäßen Verfahrens eine sogenannte Bandkalziniervorrichtung mit mindestens einer Kalzinierungszone n und einem durch die Kalzinierungszone mit im Wesentlichen konstanter Geschwindigkeit geführten Förderband ein. Bei dem Förderband handelt es sich in der Regel um ein Endlosband, das horizontal durch die Kalzinierungszone führbar ist und an seinem einen Ende eine ansteigende Umkehrbahn und an seinem anderen Ende eine absteigende Umkehrbahn aufweist. Vielfach ist es vorteilhaft, die an- und absteigende Umkehrbahn sowie den Rücklaufbereich des Fördebandes unterhalb der Kalzinierungszone mit einem Gehäuse vorzusehen. In der Nähe der ansteigenden Umkehrbahn sind zweckmäßigerweise Mittel zum Aufgeben des partikulären Katalysatorprecursors auf das Förderband und an der absteigenden Umkehrbahn Mittel zur Entnahme des kalzinierten Katalysators vom Förderband vorzusehen. Als geeignetes Mittel zum Aufgeben sei beispielsweise ein mit dem partikulärem Katalysator-precursor gefüllter Schacht zu nennen, wobei durch die Bewegung des Förderbandes eine Schüttung des Katalysatorprecursors mit im Wesentlichen konstanter Schichtdicke unter dem Schacht weggezogen wird. Vorteilhafterweise weist der Schacht zudem eine verstellbare Abstreifvorrichtung, d.h. ein sogenanntes Wehr auf, welches eine gezielte Einstellung der Schichthöhe und eine gleichmäßige Verteilung quer zur Förderrichtung erlaubt.

Die Schüttungshöhe des Katalysatorprecursors kann bei Gewährleistung eines ausreichenden Gasaustausches und der gewünschten Temperaturkonstanz im Prinzip nahezu beliebig gewählt werden. Im Allgemeinen beträgt sie im Bereich von einer Monolage der Katalysatorprecursor-Partikel bis zu 25. cm, bevorzugt 1 bis 20 cm, besonders bevorzugt 5 bis 15 cm und ganz besonders bevorzugt 8 bis 15 cm. An der absteigenden Umkehrbahn wird der kalzinierte Katalysator vom Förderband abgeworfen und durch geeignete Mittel gesammelt. Das Förderband besteht in der Regel aus einem hitzebeständigen Material, beispielsweise aus Metall.

Die Geschwindigkeit, mit der man das Förderband durch die Kalzinierzone n führt, beträgt im Allgemeinen etwa 0,1 bis 5 cm/min. Die Geschwindigkeit des Förderbandes und somit des Stromes des Katalysatorprecursors ist beim erfindungsgemäßen Verfahren im Wesentlichen konstant, wobei die zu betrachtende Zeitskala in der Größenordnung der Verweilzeit des Stromes des Katalysatorprecursors in der Kammer n liegt.

In einer bevorzugten Ausführungsform setzt man ein gasdurchlässiges Förderband ein und leitet in der Kalzinierungszone n senkrecht zur Fortbewegungsrichtung einen Gasstrom durch den Strom des Katalysatorprecursors. Das gasdurchlässige Förderband besteht beispielsweise aus einem perforierten Band, einem Gewebe oder einem Gewirke aus hitzebeständigem Material, beispielsweise aus Metall, wie etwa legiertem Stahl.

Die Kalzinierungszone n ist im Allgemeinen in Form einer sogenannten Kammer ausgebildet, d.h. von seitlichen Wandungen sowie einer oberen und einer unteren Wandung umgeben. Die in Förderrichtung des Förderbandes befindlichen Wandungen sind mit Öffnungen zur Durchführung des Stromes des Katalysatorprecursors versehen.

Die Beheizung der Kammer kann auf verschiedene Weise erfolgen. So ist es beispielsweise möglich, die erforderliche Wärme über die Zufuhr von aufgeheiztem Gas, welches z.B. zuvor aus der Kammer abgeführt und durch eine Heizquelle aufgeheizt wurde, einzubringen. Des weiteren ist es möglich, die Kammer n über die Wandungen, beispielsweise elektrisch oder über Brenner, zu beheizen. Vorzugsweise erfolgt die Beheizung durch einen unterhalb der Kammer befindlichen Brenner, welcher etwa mit Erdgas betrieben wird. Um eine möglichst gleichmäßige Verteilung der Wärme zu erreichen, ist der sogenannte Brennraum von einer weiteren Wandung umgeben und nach außen hin isoliert. Im Allgemeinen wird die Temperatur in der Kalzinierungszone n über Temperaturmeßstellen (z.B. Thermoelemente) gemessen und die Heizung entsprechend geregelt.

Da auch die umgebende Gasatmosphäre im Allgemeinen einen entscheidenden Einfluß auf die Eigenschaften des kalzinierten Katalysators hat und durch die Kalzinierung Gas aus der umgebenden Atmosphäre verbraucht werden kann sowie entstehende gasförmige Komponenten an die umgebende Atmosphäre abgegeben werden können, ist es besonders wichtig, die Gasatmosphäre durch kontinuierliche Zufuhr von Frischgas und damit verbunden auch durch kontinuierliche Abfuhr eines sogenannten Purge-Stroms, gezielt einzustellen. Je nach gewünschter Gasatmosphäre wird daher eine entsprechende Menge an dem erforderlichen Frischgas kontinuierlich zugeführt und ein entsprechender Purge-Strom kontinuierlich abgeführt. Zur Erzielung einer möglichst hohen örtlichen und zeitlichen Temperaturkonstanz sowie einer weitgehend homogenen Gasatmosphäre im Bereich des Stromes des Katalysatorprecursors wird die Atmosphäre in der Kammer der Kalzinierungszone n vorzugsweise umgewälzt.

Bevorzugt stellt man hierzu in der Kalzinierungszone n eine Gaszirkulation ein, bei der das Volumen des pro Zeiteinheit in der Kalzinierungszone n zirkulierenden Gases größer ist als das Volumen des pro Zeiteinheit in der Kalzinierungszone n frisch zugeführten Gases.

Die genannte Gaszirkulation kann dabei auf verschiedene Arten sichergestellt werden. Eine Möglichkeit besteht beispielsweise darin, Gas aus der Kammer, bevorzugt aus dem oberen Teil, abzuführen; über ein Gebläse und gegebenenfalls über einen Wärmetauscher zu führen und der Kammer, bevorzugt in den unteren Teil unterhalb des gasdurchlässigen Förderbandes, wieder zuzuführen. In einer anderen und beim erfindungsgemäßen Verfahren bevorzugten Variante weist die Kammer selbst Mittel zum Erzeugen der Gaszirkulation auf.

In einer besonders bevorzugten Ausführungsform umfassen die Mittel zur Erzeugung der Gaszirkulation einen Ventilator, der geeigneterweise oberhalb des Förderbands in der Kammer angeordnet ist. In geeigneten Ausführungsformen umfassen die Mittel zum Erzeugen der Gaszirkulation außerdem Gasleiteinrichtungen zum Leiten der Gaszirkulation innerhalb der Kammer, wobei sich die Gasleiteinrichtungen innerhalb der Kammer jeweils am Rand des Förderbands im Wesentlichen in einer Ebene senkrecht zur Auflagefläche des Förderbands erstrecken. Die Mittel zum Erzeugen der Gaszirkulation und/oder die Gasleiteinrichtungen sind zweckmäßigerweise so ausgebildet, dass das Gas durch das gasdurchlässige Förderband und die darauf befindliche Schüttung des teilchenförmigen Katalysatorprecursors aufsteigt und an den Wänden der Kammer wieder absteigt. Andererseits ist aber auch eine Gaszirkulation in umgekehrter Richtung vorstellbar. Weist die Bandkalziniervorrichtung wenigstens zwei beheizbare Kammern auf, sind diese vorzugsweise so gegeneinander abgegrenzt, dass im Wesentlichen kein Gasaustausch zwischen den Kammern stattfindet.

Beim erfindungsgemäßen Verfahren beträgt die zeitliche Schwankung der Gastemperatur vom Sollwert an jeder Stelle im Bereich des Stromes des Katalysatorprecursors nach der halben Länge der Kalzinierungszone $l_n/2$ jeweils ≤ 5°C und die lokale Differenz der Gastemperatur zwischen beliebigen Stellen im Bereich des Stromes des Katalysatorprecursors nach der halben Länge der Kalzinierungszone $l_n/2$ jeweils ≤ 5°C.

Die Temperaturkonstanz kann mittels Temperaturmeßstellen, d.h. vorzugsweise Thermoelementen, überprüft werden. Pro Kalzinierungszone n sind bevorzugt wenigstens 4, besonders bevorzugt wenigstens 6 und ganz besonders bevorzugt wenigstens 10 Temperaturmeßstellen einzusetzen, die im Bereich des Stromes des Katalysatorprecursors nach der halben Länge der Kalzinierungszone $l_n/2$ möglichst äquidistant zueinander und verteilt über die gesamte Breite des Stromes des Katalysatorprecursors angeordnet sind.

Unter der zeitlichen Schwankung der Gastemperatur vom Sollwert ist der Betrag der Differenz zwischen dem Stundenmittelwert, gemessen an einem festen Ort x im Bereich des Stromes des Katalysatorprecursors nach der halben Länge der Kalzinierungszone $l_n/2$, und dem Sollwert zu verstehen. Die zeitliche Schwankung der Gastemperatur vom Sollwert kann daher ausgedrückt werden als T(Stundenmittelwert am Ort x) - T(Sollwert)|.

Unter der lokalen Differenz der Gastemperatur ist der Betrag der Differenz zwischen dem Stundenmittelwert, gemessen an einem festen Ort x, und dem Stundenmittelwert, gemessen an einem festen Ort y, jeweils im Bereich des Stromes des Katalysatorprecursors nach der halben Länge der Kalzinierungszone $l_n/2$, zu verstehen. Die lokale Differenz der Gastemperatur kann daher ausgedrückt werden als |T (Stundenmittelwert am Ort x) - T(Stundenmittelwert am Ort y)|.

Beim erfindungsgemäßen Verfahren beträgt die zeitliche Schwankung der Gastemperatur vom Sollwert an jeder Stelle im Bereich des Stromes des Katalysatorprecursors nach der halben Länge der Kalzinierungszone $l_n/2$ bevorzugt jeweils ≤ 3°C und besonders bevorzugt jeweils ≤ 2°C.

Beim erfindungsgemäßen Verfahren beträgt die lokale Differenz der Gastemperatur zwischen beliebigen Stellen im Bereich des Stromes des Katalysatorprecursors nach der halben Länge der Kalzinierungszone $l_n/2$ bevorzugt jeweils ≤ 3°C und besonders bevorzugt jeweils ≤ 2°C.

Die Aufzeichnung der an den einzelnen Thermoelementen gemessenen Temperaturen und die Bildung der Mittelwerte erfolgen zweckmäßigerweise automatisiert durch einen entsprechend programmierten Computer. Durch diesen erfolgt vorteilhafterweise auch die Regelung bzw. Steuerung der Beheizung der Kalzinierungszonen. Es ist ratsam, die Thermoelemente regelmäßig zu kalibrieren, um zu gewährleisten, dass die maximale Abweichung der gemessenen Temperatur von der tatsächlichen.Temperatur vorzugsweise weniger als 0,5°C beträgt.

Die Gasatmosphäre in der Kalzinierungszone n enthält im Allgemeinen Sauerstoff, Stickstoff, Wasserstoffoxid (Wasserdampf), Kohlendioxid, Kohlenmoxid und/oder Edelgase. Des weiteren kann die Gasatmosphäre auch Verdampfungs- und Zersetzungsprodukte von im Katalysatorprecursor enthaltenden Komponenten, wie beispielsweise Porenbildnern oder Hilfsstoffen enthalten.

Man führt den Strom des Katalysatorprecursors zur Kalzinierung durch mindestens zwei, beispielsweise zwei bis zehn Kalzinierungszonen, wobei die Temperaturen und die Gasatmosphären der einzelnen Kalzinierungszonen voneinander unabhängig regelbar sind. Auf diese Weise lassen sich unterschiedliche Temperatur- und Gasprofile verwirklichen, die von dem Strom des Katalysatorprecursors durchlaufen werden. So können die aufeinander folgenden Kalzinierungszonen beispielsweise auf stufenweise steigende oder fallende Temperaturen thermostatiert sein. Die einzelnen Kalzinierungszonen können zudem unterschiedliche räumliche Ausdehnung aufweisen, so dass bei konstanter Geschwindigkeit des Stroms des teilchenförmigen Katalysatorvorläufers unterschiedliche Verweilzeiten in den einzelnen Kalzinierungszonen resultieren. Es ist natürlich auch möglich, benachbarte Kalzinierungszonen bei derselben Temperatur und Gasatmosphäre zu betreiben, um etwa eine längere Verweilzeit zu erhalten. Des weiteren ist es selbstverständlich auch möglich, benachbarte Kalzinierungszonen bei derselben Temperatur aber unterschiedlicher Gasatmosphäre zu betreiben.

Je nach Anzahl der insgesamt gewünschten Kalzinierungszonen und der Anzahl der Kalzinierungszonen in einer Bandkalziniervorrichtung können gegebenenfalls auch mehrere, zum Beispiel zwei oder drei, hintereinandergeschaltete Bandkalziniervorrichtungen betrieben werden. Dies kann gegebenenfalls auch vorteilhaft sein, wenn beispielsweise zur Optimierung des Kalzinierungsprozesses verschiedene Schütthöhen und/oder verschiedene Verweilzeiten erforderlich sind. Der Katalysatorprecursor kann gegebenenfalls nach dem Durchlaufen einer Bandkalziniervorrichtung und vor dem Durchlaufen einer weiteren Bandkalziniervorrichtung gesammelt und zwischengelagert werden.

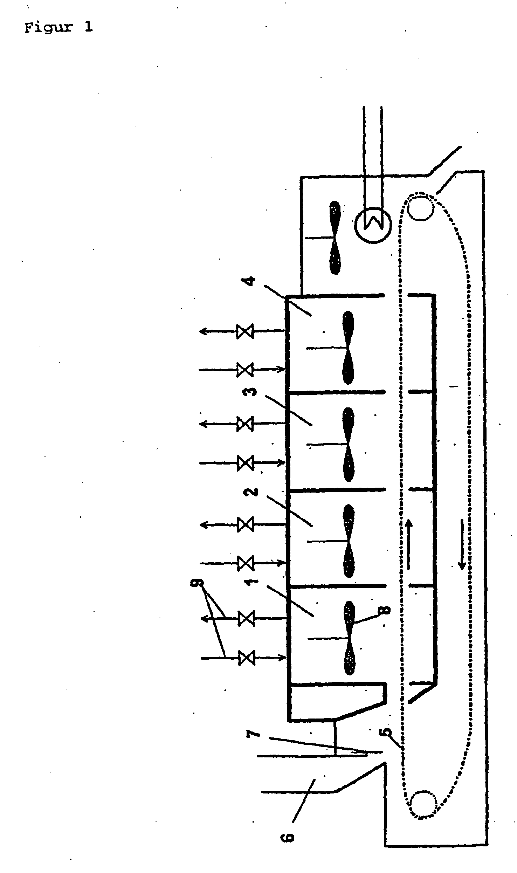

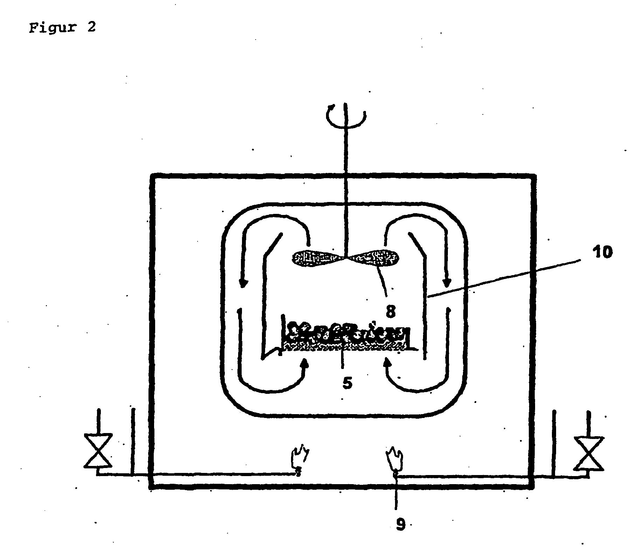

Eine bevorzugte Ausführungsform einer beim erfindungsgemäßen Verfahren bevorzugt einsetzbaren Bandkalziniervorrichtung ist schematisch durch die beiliegenden Abbildungen veranschaulicht. Fig. 1 zeigt einen Längsschnitt, Fig. 2 einen Querschnitt durch die bevorzugte Bandkalziniervorrichtung.

Mit Bezug auf Fig. 1 weist die dargestellte Bandkalziniervorrichtung vier umkammerte Kalzinierungszonen auf (1, 2, 3, 4), durch die ein gasdurchlässiges Förderband (5) läuft. Die Bandkalziniervorrichtung umfasst ferner einen Bunker (6) mit einem verstellbaren Wehr (7). Oberhalb des Förderbandes (5) befinden sich in jeder Kammer ein oder mehrere Ventilator (en) (8). Jede Kammer ist mit Zu- und Ablufteinrichtungen (9) versehen. Im Betrieb ist der Bunker (6) mit dem teilchenförmigen Katalysatorprecursor gefüllt. Durch die Bewegung des Förderbandes (5) wird unter dem Wehr (7) eine Schicht des Katalysatorprecursors mit konstanter Schütthöhe weggezogen und durchläuft nacheinander die Kammern der Bandkalziniervorrichtung.

Mit Bezug auf Fig. 2 wird jede Kammer durch einen oder mehrere Brenner (9) beheizt. An den Rändern des Förderbands sind im Wesentlichen in einer Ebene senkrecht zur Auflagefläche des Förderbands Gasleitbleche (10) angeordnet, die zusammen mit den Ventilatoren (8) dafür sorgen, dass die umgewälzte Atmosphäre in jeder Kammer durch das gasdurchlässige Förderband (5) aufsteigt und an den Wänden der Kammer wieder absteigt.

Die beim erfindungsgemäßen Verfahren einsetzbaren Katalysatorprecursor umfassen als katalytisch aktive Masse eine sauerstoffhaltige Vanadium-Phosphor-Verbindung oder Gemische solcher Verbindungen. Geeignete Aktivmassen sind beispielsweise in den Patentschriften US 5,275,996, US 5,641,722, US 5,137,860, US 5,095,125 oder US 4,933,312 beschrieben.

Sie können des weiteren sogenannte Promotoren enthalten. Als geeignete Promotoren sind die Elemente der 1. bis 15. Gruppe des Periodensystems sowie deren Verbindungen genannt. Geeignete Promotoren sind beispielsweise in den Offenlegungsschriften WO 97/12674 und WO 95/26817 sowie in den Patenten US 5,137,860, US 5,296,436, US 5,158,923 und US 4,795,818 beschrieben. Bevorzugt werden als Promotoren Verbindungen der Elemente Kobald, Molybdän, Eisen, Zink, Hafnium, Zirkon, Lithium, Titan, Chrom, Mangan, Nickel, Kupfer; Bor, Silicium, Antimon, Zinn, Niob und Wismut, besonders bevorzugt Molybdän, Eisen, Zink, Antimon, Wismut, Lithium. Die promotierten Katalysatoren können einen oder mehrere Promotoren enthalten. Der Gehalt an Promotoren beträgt in Summe im fertigen Katalysator im Allgemeinen nicht mehr als etwa 5 Gew.-%, jeweils als Oxid gerechnet.

Durch die vorangehende Herstellung des Katalysatorprecusors kann dieser ferner auch sogenannte Hilfsmittel, wie etwa Tablettierhilfsmittel oder Porenbildner enthalten.

Tablettierhilfsmittel werden im Allgemeinen zugesetzt, wenn die Formgebung der erfindungsgemäßen Katalysatoren über eine Tablettierung erfolgt. Tablettierhilfsmittel sind in der Regel katalytisch inert und verbessern die Tablettiereigenschaften des sogenannten Precursorpulvers, einer Zwischenstufe in der Katalysatorherstellung, beispielsweise durch Erhöhung der Gleit- und Rieselfähigkeit. Als geeignetes und bevorzugtes Tablettierhilfsmittel sei Graphit genannt. Die zugesetzten Tablettierhilfsmittel verbleiben in der Regel im aktivierten Katalysator. Typischerweise liegt der Gehalt an Tablettierhilfsmittel im fertigen Katalysator bei etwa 2 bis 6 Gew.-%.

Porenbildner sind Stoffe, welche zur gezielten Einstellung der Porenstruktur im Makroporenbereich eingesetzt werden. Sie können prinzipiell unabhängig vom Formgebungsverfahren eingesetzt werden. In der Regel handelt es sich um Kohlenstoff, Wasserstoff, Sauerstoff und/oder Stickstoff enthaltende Verbindungen, welche vor der Formgebung des Katalysator zugesetzt werden und bei der anschließenden Aktivierung des Katalysators unter Sublimation, Zersetzung und/oder Verdampfung zum überwiegenden Teil wieder entfernt werden. Der fertige Katalysator kann dennoch Rückstände oder Zersetzungsprodukte des Porenbildners enthalten.

Die beim erfindungsgemäßen Verfahren einsetzbaren Katalysatorprecursor können die Aktivmasse beispielsweise in reiner, unverdünnter Form als sogenannter "Vollkatalysator-Precusor" oder verdünnt mit einem bevorzugt oxidischen Trägermaterial als sogenannter "Mischkatalysator-Precursor" enthalten. Als geeignete Trägermaterialien für Mischkatalysatoren seien beispielsweise Aluminiumoxid, Siliciumdioxid, Alumosilikate, Zirkondioxid, Titandioxid oder Gemische davon genannt. Bevorzugt ist die Herstellung von Voll- und Mischkatalysatoren, besonders bevorzugt von Vollkatalysatoren.

Der beim erfindungsgemäßen Verfahren einsetzbare Katalysatorprecursor weist Partikel mit einem gemittelten Durchmesser von mindestens 2 mm, bevorzugt mindestens 3 mm auf. Unter dem gemittelten Durchmesser eines Partikels ist der Mittelwert aus der kleinsten und der größten Abmessung zwischen zwei planparallelen Platten zu verstehen.

Unter Partikeln sind sowohl regellos geformte Partikel als auch geometrisch geformte Partikel, sogenannte Formkörper, zu verstehen. Bevorzugt weist der beim erfindungsgemäßen Verfahren einzusetzende Katalysatorprecursor Formkörper auf. Als geeignete Formkörper seien beispielsweise genannt Tabletten, Zylinder, Hohlzylinder, Kugeln, Stränge, Wagenräder oder Extrudate. Besondere Formen, wie beispielsweise "Trilobes" und "Tristars" (siehe EP-A-0 593 646) oder Formkörper mit mindestens einer Einkerbung an der Außenseite (siehe US 5,168,090) sind ebenfalls möglich.

Besonders bevorzugt weist der beim erfindungsgemäßen Verfahren einsetzbare Katalysatorprecursor Formkörper mit einer im Wesentlichen hohlzylinderförmigen Struktur auf. Unter einer im wesentlichen hohlzylinderförmigen Struktur ist eine Struktur zu verstehen, welche im wesentlichen einen Zylinder mit einer zwischen den beiden Deckelflächen hindurchgehenden Öffnung umfaßt. Der Zylinder ist charakterisiert durch zwei im Wesentlichen parallele Deckelflächen und einer Mantelfläche, wobei der Querschnitt des Zylinders, d.h. parallel zu den Deckelflächen, im wesentlichen von kreisförmiger Struktur ist. Der Querschnitt der hindurchgehenden Öffnung, d.h. parallel zu den Deckelflächen des Zylinders, ist im wesentlichen ebenfalls von kreisförmiger Struktur. Bevorzugt befindet sich die hindurchgehende Öffnung mittig zu den Deckelflächen, wobei andere räumliche Anordnungen damit nicht ausgeschlossen sind.

Der Begriff "im Wesentlichen" weißt darauf hin, dass Abweichungen von der Idealgeometrie, wie beispielsweise leichte Deformationen der kreisförmigen Struktur, nicht planparallel ausgerichtete Dekkelflächen, abgeplatzte Ecken und Kanten, Oberflächenrauhigkeit oder Einkerbungen in der Mantelfläche, den Deckelflächen oder der Innenfläche der hindurchgehenden Bohrung beim Katalysatorprecursor mit umfasst sind. Im Rahmen der Genauigkeit der Tablettierkunst sind kreisförmige Deckelflächen, ein kreisförmiger Querschnitt der hindurchgehenden Bohrung, parallel ausgerichtete Dekkelflächen und makroskopisch glatte Oberflächen bevorzugt.

Die im Wesentlichen hohlzylinderförmige Struktur kann beschrieben werden durch einen äußeren Durchmesser $d_1$, einer Höhe h als Abstand der beiden Deckelflächen und einem Durchmesser des inneren Lochs (hindurchgehende Öffnung) $d_2$. Der äußere Durchmesser $d_1$ des Katalysatorprecursors beträgt bevorzugt 3 bis 10 mm, besonders bevorzugt 4 bis 8 mm, ganz besonders bevorzugt 4,5 bis 6 mm. Die Höhe h beträgt bevorzugt 1 bis 10 mm, besonders bevorzugt 2 bis 6 mm, ganz besonders bevorzugt 2 bis 3,5 mm. Der Durchmesser der hindurchgehenden Öffnung $d_2$ beträgt bevorzugt 1 bis 8 mm, besonders bevorzugt 2 bis 6 mm, ganz besonders bevorzugt 2 bis 3 mm.

Die beim erfindungsgemäßen Verfahren einsetzbare Katalysatorprecursor kann beispielsweise wie in den Patentschriften US 5,275,996 und US 5,641,722 oder der Offenlegungsschrift WO 97/12674 beschrieben hergestellt werden. Die wesentlichen Schritte einer bevorzugten Herstellung des Katalysatorprecursors sind im Folgenden erläutert.

(a) Umsetzung einer fünfwertigen Vanadiumverbindung (z.B. $V_2O_5$) und gegebenenfalls einer Promotorkomponente (z.B. $MoO_3$) mit einem organischen, reduzierenden Lösungsmittel (z.B. Alkohol, wie etwa Isobutanol) in Gegenwart einer fünfwertigen Phosphorverbindung (z.B. Ortho- und/oder Pyrophosphorsäure, Phosphorsäureester) und/oder einer dreiwertigen Phosphorverbindung (z.B. phosphorige Säure) unter Erwärmen. Gegebenenfalls kann diese Stufe in Gegenwart eines dispergierten, pulverförmigen Trägermaterials durchgeführt werden. Bevorzugt ist die Umsetzung ohne Zusatz von Trägermaterial.

(b) Isolierung des gebildeten Vanadium-, Phosphor-, Sauerstoff und gegebenenfalls Promotor enthaltenden Katalysatorprecursors ("VPO-Precursor"), z.B. durch Filtration oder Eindampfung.

(c) Trocknung des VPO-Precursors und bevorzugt beginnende Präformierung durch Temperung bei einer Temperatur von 250 bis 350°C. Dem getrockneten und bevorzugt getemperten VPO-Precursor-Pulver kann nun gegebenenfalls pulverförmiges Trägermaterial und/oder ein sogenannter Porenbildner, wie beispielsweise Stearinsäure, Cellulose oder Paraffine untermischt werden. Bevorzugt ist die Weiterverarbeitung ohne Zusatz eines Trägermaterials sowie ohne Zusatz eines Porenbildners.

(d) Formgebung durch Überführung in die gewünschte Struktur, bevorzugt in die im Wesentlichen hohlzylinderförmige Struktur. Die Formgebung erfolgt bevorzugt durch Tablettierung, vorteilhafterweise unter vorheriger Untermischung eines sogenannten Gleitmittels, wie etwa Graphit.

Als weniger bevorzugte Alternative zur Tablettierung sei beispielsweise die Extrusion genannt. Bei dieser Variante teigt man beispielsweise den in (b) erhaltenen VPO-Precursor an, um eine extrusionsfähige Masse zu erhalten. Diese kann dann in die gewünschte Struktur extrutiert und zum Erhalt des einsetzbaren Katalysatorprecursors getrocknet werden.

Die Kalzinierung des Katalysatorprecursors erfolgt beim erfindungsgemäßen Verfahren in Gegenwart einer Atmosphäre, enthaltend Sauerstoff, Wasserstoffoxid (Wasserdampf) und/oder Inertgas in einem Temperaturbereich von 250 bis 600°C. Als geeignete Inertgase seien beispielsweise Stickstoff, Kohlendioxid 'und Edelgase genannt. Bevorzugt werden bei der Kalzinierung nach dem erfindungsgemäßen Verfahren mindestens zwei Kalzinierungszonen, beispielsweise zwei bis zehn Kalzinierungszonen, mit unterschiedlicher Gasatmosphäre und gegebenenfalls unterschiedlicher Temperatur durchlaufen. Durch geeignete, an das jeweilige Katalysatorsystem angepaßte Kombination von Temperaturen, Behandlungsdauern und Gasatmosphären kann die mechanische und katalytische Eigenschaft des Katalysators beeinflußt und somit gezielt eingestellt werden.

Beim erfindungsgemäßen Verfahren erfolgt eine Kalzinierung, bei der man den Katalysatorprecursor

(a) in mindestens einer Kalzinierungszone in einer oxidierenden Atmosphäre mit einem Sauerstoff-Gehalt von 2 bis 21 Vol.-% auf eine Temperatur von 200 bis 350°C aufheizt und unter diesen Bedingungen bis zur Einstellung der gewünschten mittleren Oxidationsstufe des Vanadiums beläßt; und

(b) in mindestens einer weiteren Kalzinierungszone in einer nicht-oxidierenden Atmosphäre mit einem Sauerstoff-Gehalt von $\leq$ 0,5 Vol.-% und einem Wasserstoffoxid-Gehalt von 20 bis 75 Vol.-% auf eine Temperatur von 300 bis 500°C aufheizt und $\geq$ 0,5 Stunden unter diesen Bedingungen beläßt.

Bei Schritt (a) wird der Katalysatorprecursor in einer oxidierend wirkenden Atmosphäre mit einem Gehalt an molekularem Sauerstoff von im Allgemeinen 2 bis 21 Vol.-% und bevorzugt von 5 bis 21 Vol.-% bei einer Temperatur von 200 bis 350°C und bevorzugt von 250 bis 350°C über einen Zeitraum, der wirksam ist, die gewünschte mittlere Oxidationsstufe des Vanadiums einzustellen, belassen. Im Allgemeinen setzt man bei Schritt (a) Mischungen aus Sauerstoff, Inertgasen (z.B. Stickstoff oder Argon), Wasserstoffoxid (Wasserdampf) und/oder Luft sowie Luft ein. Aus der Sicht des durch die Kalzinierungszone(n) geführten Katalysatorprecursors kann die Temperatur während des Kalzinierschrittes (a) konstant gehalten werden, im Mittel steigen oder fallen. Da dem Schritt (a) im Allgemeinen eine Aufheizphase vorangeschaltet ist, wird die Temperatur in der Regel zunächst ansteigen, um dann bei dem gewünschten Endwert einzupendeln. Im Allgemeinen ist daher der Kalzinierungszone von Schritt' (a) mindestens eine weitere Kalzinierungszone zur Aufheizung des Katalysatorprecursors vorangeschaltet.

Der Zeitraum, über den die Temperung in Schritt (a) aufrecht erhalten wird, ist beim erfindungsgemäßen Verfahren bevorzugt derart zu wählen, dass sich eine mittlere Oxidationsstufe des Vanadiums auf einen Wert von +3,9 bis +4,4, bevorzugt von +4,0 bis +4,3, einstellt. Die Bestimmung der mittleren Oxidationsstufe des Vanadiums erfolgt über potentiometrische Titration nach der in den Beispielen beschriebenen Methode.

Da die Bestimmung der mittleren Oxidationsstufe des Vanadiums während der Kalzinierung aus apparativen und zeitlichen Gründen nur äußerst schwierig zu bestimmen ist, ist der erforderliche Zeitraum vorteilhafterweise in Vorversuchen experimentell zu bestimmen. In der Regel dient hierzu eine Meßreihe, bei der unter definierten Bedingungen getempert wird, wobei die Proben nach un-terschiedlichen Zeiten aus dem System entfernt, abgekühlt und bezüglich der mittleren Oxidationsstufe des Vanadiums analysiert werden.

Der bei Schritt (a) erforderliche Zeitraum ist im Allgemeinen abhängig von der Natur des Katalysatorprecursors, der eingestellten Temperatur und der gewählten Gasatmosphäre, insbesondere des Sauerstoff-Gehalts. Im Allgemeinen erstreckt sich der Zeitraum bei Schritt (a) auf eine Dauer von über 0,5 Stunden und bevorzugt von über 1 Stunde. Im Allgemeinen ist ein Zeitraum von bis zu 4 Stunden, bevorzugt von bis zu 2 Stunde zur Einstellung der gewünschten mittleren Oxidationsstufe ausreichend. Unter entsprechend eingestellten Bedingungen (z.B. unterer Bereich des Temperaturintervalls und/oder geringer Gehalt an molekularem Sauerstoff) kann aber auch ein Zeitraum von über 6 Stunden erforderlich sein.

Bei Schritt (b) wird die erhaltene Katalysatorzwischenstufe in einer nicht-oxidierenden Atmosphäre mit einem Gehalt an molekularem Sauerstoff von $\leq$ 0,5 Vol.-% und an Wasserstoffoxid (Wasserdampf) von 20 bis 75 Vol.-%, bevorzugt von 30 bis 60 Vol.-% bei einer Temperatur von 300 bis 500°C und bevorzugt von 350 bis 450°C über einen Zeitraum von $\geq$ 0,5 Stunden, bevorzugt 2 bis 10 Stunden und besonders bevorzugt 2 bis 4 Stunden belassen. Die nicht-oxidierende Atmosphäre enthält neben dem genannten Wasserstoffoxid im Allgemeinen überwiegend Stickstoff und/oder Edelgase, wie beispielsweise Argon, wobei hierunter keine Einschränkung zu verstehen ist. Auch Gase, wie beispielsweise Kohlendioxid sind prinzipiell geeignet. Bevorzugt enthält die nicht-oxidierende Atmosphäre $\geq$ 40 Vol.-% Stickstoff. Aus der Sicht des durch die Kalzinierungszone(n) geführten Katalysatorprecursors kann die Temperatur während des Kalzinierschrittes (b) konstant gehalten werden, im Mittel steigen oder fallen. Wird Schritt (b) bei einer höheren oder tieferen Temperatur als Schritt (a) durchgeführt, so befindet sich zwischen den Schritten (a) und (b) in der Regel eine Aufheiz- oder Abkühlphase, welche gegebenenfalls in einer weiteren Kalzinierungszone implementiert ist. Um eine verbesserte Trennung zur sauerstoffhaltigen Atmosphäre des Schrittes (a) zu ermöglichen, kann diese weitere Kalzinierungszone zwischen (a) und (b) beispielsweise zur Spülung mit Inertgas, wie beispielsweise Stickstoff, gespült werden. Bevorzugt wird Schritt (b) bei einer um 50 bis 150 höheren Temperatur als Schritt (a) durchgeführt.

Im Allgemeinen umfasst die Kalzinierung einen weiteren, zeitlich nach Schritt (b) durchzuführenden Schritt (c), bei dem man den kalzinierten Katalysatorprecursor in einer Inertgas-Atmosphäre auf eine Temperatur von $\leq 300°C$, bevorzugt von $\leq 200°C$ und besonders bevorzugt von $\leq 150°C$ abkühlt.

Vor, zwischen und/oder nach den Schritten (a) und (b), beziehungsweise (a), (b) und (c) sind bei der Kalzinierung nach dem erfindungsgemäßen Verfahren weitere Schritte möglich. Ohne limitierend zu wirken seien als weitere Schritte beispielsweise Änderungen in der Temperatur (Aufheizen, Abkühlen), Änderungen in der Gasatmosphäre (Umstellung der Gasatmosphäre), weitere Haltezeiten, Überführungen der Katalysatorzwischenstufe in andere Apparate oder Unterbrechungen des gesamten Kalziniervorgangs genannt.

Da der Katalysator-Precursor in der Regel vor Beginn der Kalzinierung eine Temperatur von $< 100°C$ besitzt, ist dieser vor Schritt (a) üblicherweise aufzuheizen. Das Aufheizen kann unter Anwendung verschiedener Gasatmosphären durchgeführt werden. Vorzugsweise wird das Aufheizen in einer oxidierend wirkenden Atmosphäre, wie unter Schritt (a) definiert, oder einer Inertgas-Atmosphäre, wie unter Schritt (c) definiert, durchgeführt. Auch ein Wechsel der Gasatmosphäre während der Aufheizphase ist möglich. Besonders bevorzugt ist das Aufheizen in der oxidierend wirkenden Atmosphäre, welche auch in Schritt (a) angewendet wird.

Der durch das erfindungsgemäße Verfahren bevorzugt hergestellte Katalysator weist ein Phosphor/Vanadium-Atomverhältnis von 0,9 bis 1,5, besonders bevorzugt von 0,9 bis 1,2 und ganz besonders bevorzugt von 1,0 bis 1,1, eine mittlere Oxidationsstufe des Vanadiums von +3,9 bis +4,4 und besonders bevorzugt von 4,0 bis 4,3, eine BET-Oberfläche von 10 bis 50 $m^2/g$ und besonders bevorzugt von 20 bis 40 $m^2/g$, ein Porenvolumen von 0,1 bis 0,5 ml/g und besonders bevorzugt von 0,2 bis 0,4 ml/g und eine Schüttdichte von 0,5 bis 1,5 kg/l und besonders bevorzugt 0,5 bis 1,0 kg/l auf.

Weiterhin ist ein Verfahren zur Herstellung von Maleinsäureanhydrid durch heterogenkatalytische Gasphasenoxidation eines Kohlenwasserstoffs mit mindestens vier Kohlenstoffatomen mit Sauerstoff enthaltenden Gasen, dadurch gekennzeichnet, dass man einen erfindungsgemäßen Katalysator gemäß obiger Beschreibung einsetzt.

Beim Verfahren zur Herstellung von Maleinsäureanhydrid werden im Allgemeinen Rohrbündelreaktoren eingesetzt. Als Kohlenwasserstoffe werden im Allgemeinen aliphatische und aromatische, gesättigte und ungesättigte Kohlenwasserstoffe mit mindestens vier Kohlenstoffatomen, wie beispielsweise 1,3-Butadien, 1-Buten, 2-cis-Buten, 2-trans-Buten, n-Butan, $C_4$-Gemisch, 1,3-Pentadien, 1,4-Pentadien, 1-Penten, 2-cis-Penten, 2-trans-Penten, n-Pentan, Cyclopentadien, Dicyclopentadien, Cyclopenten, Cyclopentan, $C_5$-Gemisch, Hexene, Hexane, Cyclohexan und Benzol geeignet. Bevorzugt eingesetzt werden 1-Buten, 2-cis-Buten, 2-trans-Buten, n-Butan, Benzol oder deren Mischungen geeignet. Besonders bevorzugt ist der Einsatz von n-Butan und n-Butan-haltigen Gasen und Flüssigkeiten. Das verwendete n-Butan kann beispielsweise aus dem Erdgas, aus Steamcrackern oder FCC-Crackern stammen.

Die Zugabe des Kohlenwasserstoffs erfolgt im Allgemeinen mengengeregelt, d.h. unter stetiger Vorgabe einer definierten Menge pro Zeiteinheit. Der Kohlenwasserstoff kann in flüssiger oder gasförmiger Form dosiert werden. Bevorzugt ist die Dosierung in flüssiger Form mit anschließender Verdampfung vor Eintritt in den Rohrbündelreaktor.

Als Oxidationsmittel werden Sauerstoff enthaltende Gase, wie beispielsweise Luft, synthetische Luft, ein mit Sauerstoff angereichertes Gas oder auch sogenannter "reiner", d.h. z.B. aus der Luftzerlegung stammender Sauerstoff eingesetzt. Auch das Sauerstoff-enthaltende Gas wird mengengeregelt zugegeben.

Das durch den Rohrbündelreaktor zu leitende Gas enthält im Allgemeinen eine Kohlenwasserstoff-Konzentration von 0,5 bis 15 Vol.-% und eine Sauerstoff-Konzentration von 8 bis 25 Vol.-%. Der zu einhundert Vol.-% fehlende Anteil setzt sich aus weiteren Gasen wie beispielsweise Stickstoff, Edelgasen, Kohlenmonoxid, Kohlendioxid, Wasserdampf, oxygenierte Kohlenwasserstoffe (z.B. Methanol, Formaldehyd, Ameisensäure, Ethanol, Acetaldehyd, Essigsäure, Propanol, Propionaldehyd, Propionsäure, Acrolein, Crotonaldehyd) und deren Mischungen zusammen. Bevorzugt beträgt der n-Butan-Anteil an der Gesamtmenge an Kohlenwasserstoff $\geq 90\%$ und besonders bevorzugt $\geq 95\%$.

Zur Gewährung einer langen Katalysatorstandzeit und weiterer Erhöhung von Umsatz, Selektivität, Ausbeute, Katalysator-Belastung und Raum/Zeit-Ausbeute wird dem Gas beim erfindungsgemäßen Verfahren bevorzugt eine flüchtige Phosphorverbindung zugeführt. Ihre Konzentration beträgt zu Beginn, d.h. am Reaktoreingang, mindestens 0,2 Volumen-ppm, d.h. $0,2 \cdot 10^{-6}$ Volumenanteile der flüchtigen Phosphorverbindungen bezogen auf das Gesamtvolumen des Gases am Reaktoreingang. Bevorzugt ist ein Gehalt von 0,2 bis 20 Volumen-ppm, besonders bevorzugt von 0,5 bis 10 Volumen-ppm. Als flüchtige Phosphorverbindungen sind all jene Phosphor-enthaltende Verbindungen zu verstehen, welche in der gewünschten Konzentration unter den Einsatzbedingungen gasförmig vorliegen. Als geeignete flüchtige Phosphorverbindungen sind beispielsweise Phosphine und Phosphorsäureester genannt. Besonders bevorzugt sind die $C_1$- bis $C_4$-Alkyl-Phosphorsäureester, ganz besonders bevorzugt Trimethylphosphat, Triethylphosphat und Tripropylphosphat, insbesondere Triethylphosphat.

Das Verfahren wird im Allgemeinen bei einer Temperatur von 350 bis 480°C durchgeführt. Unter der genannten Temperatur wird die Temperatur der im Rorbündelreaktor befindlichen Katalysatorschüttung verstanden, welche bei Ausübung des Verfahrens in Abwesenheit einer chemischen Reaktion vorliegen würde. Ist diese Temperatur nicht an allen Stellen exakt gleich, so meint der Begriff den Zahlenmittelwert der Temperaturen längs der Reaktionszone. Insbesondere bedeutet dies, daß die wahre, am Katalysator vorliegende Temperatur aufgrund der Exothermie der Oxidationsreaktion auch außerhalb des genannten Bereichs liegen kann. Bevorzugt wird das erfindungsgemäße Verfahren bei einer Temperatur von 380 bis 460°C, besonders bevorzugt 380 bis 430°C durchgeführt.

Das erfindungsgemäße Verfahren kann bei einem Druck unterhalb von Normaldruck (z.B. bis 0,05 MPa abs) als auch oberhalb von Normaldruck (z.B. bis 10 MPa abs) ausgeübt werden. Darunter ist der in der Rohrbündelreaktor-Einheit vorliegende Druck zu verstehen. Bevorzugt ist ein Druck von 0,1 bis 1,0 MPa abs, besonders bevorzugt 0,1 bis 0,5 MPa abs.

Das Verfahren kann in zwei bevorzugten Verfahrensvarianten, der Variante mit "geradem Durchgang" und der Variante mit "Rückführung" durchgeführt werden. Beim "geraden Durchgang" wird aus dem Reaktoraustrag Maleinsäureanhydrid und gegebenenfalls oxygenierte Kohlenwasserstoff-Nebenprodukte entfernt und das verbleibende Gasgemisch ausgeschleust und gegebenenfalls thermisch verwertet. Bei der "Rückführung" wird aus dem Reaktoraustrag ebenfalls Maleinsäureanhydrid und gegebenenfalls oxygenierte Kohlenwasserstoff-Nebenprodukte entfernt, das verbleibende Gasgemisch, welches nicht-umgesetzten Kohlenwasserstoff enthält, ganz oder teilweise zum Reaktor rückgeführt. Eine weitere Variante der "Rückführung" ist die Entfernung des nicht-umgesetzten Kohlenwasserstoffs und dessen Rückführung zum Reaktor.

In einer besonders bevorzugten Ausführungsform zur Herstellung von Maleinsäureanhydrid setzt man n-Butan als Ausgangs-Kohlenwasserstoff ein und führt die heterogenkatalytische Gasphasenoxidation "geraden Durchgang" an dem erfindungsgemäßen Katalysator durch.

In einer besonders bevorzugten Ausführungsform des erfindungsgemäßen Verfahrens zur Herstellung eines für die heterogenkatalytische Gasphasenoxidation eines Kohlenwasserstoffs mit mindestens vier Kohlenstoffatomen zu Maleinsäureanhydrid geeigneten, Vanadium, Phosphor und Sauerstoff enthaltenden Katalysators, setzt man einen Vanadylhydrogenphosphat-Hemihydrat ($VOHPO_4 \cdot \frac{1}{2} H_2O$) enthaltenden Katalysatorprecursor ein, welcher durch Erhitzen einer Vanadium(V)-Verbindung, bevorzugt Vanadiumpentoxid und Phosphorsäure, bevorzugt 100 bis 110%-ige Phosphorsäure, unter Rückfluß, Abfiltrieren, Waschen und Trocknen des entstandenen Niederschlages, Behandlung bei einer Temperatur im Bereich von 80 bis 350°C, bevorzugt von 200 bis 250°C, unter sauerstoffhaltiger Atmosphäre über einen Zeitraum von 1 bis 8 Stunden und Tablettierung in die gewünschte Form, erhalten wurde.

Der Katalysatorprecursor wird nun einer Bandkalziniervorrichtung zugeführt und über eine entsprechende Dosiereinrichtung mit verstellbaren Wehr in einer konstanten Schütthöhe im Bereich von 5 bis 15 cm auf ein gasdurchlässiges Förderband gegeben. Das gasdurchlässige Förderband wird dabei mit einer im Wesentlichen konstanten Geschwindigkeit im Bereich von 0,1 bis 5 cm/min durch die einzelnen Kalzinierungszonen geführt. Die bevorzugt eingesetzte Bandkalziniervorrichtung umfasst mindestens 5, insbesondere 6 bis 8 Kalzinierzonen, welche bei unterschiedlichen Temperaturen und/oder Gasatmosphären betrieben werden. Jede Kalzinierzone ist umkammert und mit einem oder mehreren Ventilator(en) sowie einer Zu- und Ablufteinrichtung ausgestattet. Frischgas wird kontinuierlich zu- und eine entsprechende Menge an Purge-Gas kontinuierlich abgeführt. Das Volumen des pro Zeiteinheit in der Kalzinierungszone n zirkulierenden Gases ist jeweils größer ist als das Volumen des pro Zeiteinheit in der Kalzinierungszone n zugeführten Gases.

Die Parameterbereiche, bei denen die bevorzugt eingesetzte Bandkalziniervorrichtung mit 8 Kalzinierzonen bevorzugt betrieben wird, sind in Tabelle 1 dargestellt.

Tabelle 1:

| Parameterbereiche für die bevorzugte Kalzinierung. | | | |
|---|---|---|---|
| Zone | Temperatur | zugeführtes Frischgas | mittlere Verweilzeit |
| Kalzinierzone 1 | Aufheizen auf eine Temperatur im Bereich von 150 bis 250°C | Luft oder Luft/$N_2$ | 1 bis 3 Stunden |
| Kalzinierzone 2 | Tempern bei 150 bis 250°C | Luft oder Luft/$N_2$ | 1 bis 3 Stunden |
| Kalzinierzone 3 | Tempern bei 150 bis 250°C | Luft oder Luft/$N_2$ | 1 bis 3 Stunden |
| Kalzinierzone 4 | Aufheizen auf eine Temperatur im Bereich von 250 bis 350°C | Luft oder Luft/$N_2$ | 1 bis 3 Stunden |
| Kalzinierzone 5 | Aufheizen auf Temperatur im Bereich von 250 bis 450°C | $N_2$ | 1 bis 3 Stunden |
| Kalzinierzone 6 | Tempern bei 350 bis 450°C | $N_2$/$H_2O$-Dampf | 1 bis 3 Stunden |
| Kalzinierzone 7 | Tempern bei 350 bis 450°C | $N_2$/$H_2O$-Dampf | 1 bis 3 Stunden |
| Kalzinierzone 8 | Abkühlen auf Raumtemperatur | $N_2$ | 1 bis 3 Stunden |

Beim Einsatz einer Bandkalziniervorrichtung mit 5, 6 oder 7 Kalzinierzonen sind die obengenannten Schritte 1+2, 2+3, 1+2+3 und/oder 6+7 zusammengefasst.

Bevorzugt beträgt die zeitliche Schwankung der Gastemperatur vom Sollwert an jeder Stelle im Bereich des Stromes des Katalysatorprecursors nach der halben Länge der Kalzinierungszone $l_n/2$ in den Kalzinierungszonen 2, 3, 4, 6 und 7 und besonders bevorzugt in allen Kalzinierungszonen jeweils $\leq 5°C$ und die lokale Differenz der Gastemperatur zwischen beliebigen Stellen im Bereich des Stromes des Katalysatorprecursors nach der halben Länge der Kalzinierungszone $l_n/2$ in den Kalzinierungszonen 2, 3, 4, 6 und 7 und besonders bevorzugt in allen Kalzinierungszonen jeweils $\leq 5°C$.

Das erfindungsgemäße Verfahren ermöglicht die qualitativ hochwertige Herstellung eines partikulären Vanadium, Phosphor und Sauerstoff enthaltenden Katalysators für die heterogenkatalytische Gasphasenoxidation eines Kohlenwasserstoffs mit mindestens vier Kohlenstoffatomen zu Maleinsäureanhydrid, welches, ausgehend von einem partikulären Vanadium, Phosphor und Sauerstoff enthaltenden Katalysatorprecursor, kontinuierlich durchführbar ist und auch bei der Produktion größerer Mengen im Tonnen-Maßstab, über die gesamte Produktion hinweg, zu einem sehr gleichmäßigen Katalysator mit sehr geringer Streuung der katalytischen, mechanischen, physikalischen und chemischen Eigenschaften führt. Eingesetzt in einem Rohrbündelreaktor führt der erfindungsgemäß hergestellte Katalysator zu einer sehr geringen Streuung der sich in den einzelnen Rohren einstellenden Temperaturen und ermöglicht somit eine weitergehende Optimierung hinsichtlich eines hohen Umsatzes, einer hohen Kohlenwasserstoff-Belastung, einer hohen Selektivität, einer hohen Ausbeute und einer langen Katalysatorlebensdauer bei gleichzeitig hoher Sicherheit in Bezug auf das Durchgehen der Reaktion.

Definitionen

Die in dieser Schrift verwendeten Größen sind, falls nicht anders erwähnt, wie folgt definiert:

$$\text{Raum/Zeit-Ausbeute} = \frac{m_{Maleinsäureanhydrid}}{V_{Katalysator} \cdot t}$$

$$\text{Belastung} = \frac{V_{Kohlenwasserstoff}}{V_{Katalysator} \cdot t}$$

$$\text{Umsatz } U = \frac{n_{KW,Reaktor,ein} - n_{KW,Reaktor,aus}}{n_{KW,Reaktor,ein}}$$

$$\text{Selektivität } S = \frac{n_{MSA,Reaktor,aus}}{n_{KW,Reaktor,ein} - n_{KW,Reaktor,aus}}$$

$$\text{Ausbeute } A = U \cdot S$$

| | |
|---|---|
| $m_{Maleinsäureanhydrid}$ | Masse an produziertem Maleinsäureanhydrid [g] |
| $V_{Katalysator}$ | Schüttvolumen Katalysator, summiert über alle Reaktidnszonen [l] |
| $t$ | Zeiteinheit [h] |
| $V_{Kohlenwasserstoff}$ | auf 0°C und 0,1013 MPa normiertes Volumen des Kohlenwasserstoffs in der Gasphase [N1] (Rechnerische Größe. Liegt ein Kohlenwasserstoff unter diesen Bedingungen in der Flüssigphase vor, so wird über das ideale Gasgesetz das hypothetische Gasvolumen berechnet.) |
| $U$ | Umsatz an Kohlenwasserstoffen pro Reaktordurchgang |
| $S$ | Selektivität bzgl. Maleinsäureanhydrid pro Reaktordurchgang |
| $A$ | Ausbeute an Maleinsäureanhydrid pro Reaktordurchgang |
| $n_{KW,\,Reaktor,\,ein}$ | Stoffmengenstrom an Kohlenwasserstoffen am Reaktoreingang [mol/h] |

| | |
|---|---|
| $n_{KW, Reaktor, aus}$ | Stoffmengenstrom an Kohlenwasserstoffen am Reaktorausgang [mol/h] |
| $n_{KW, Anlage, ein}$ | Stoffmengenstrom an Kohlenwasserstoffen am Eingang der Anlage [mol/h] |
| $n_{KW, Anlage, aus}$ | Stoffmengenstrom an Kohlenwasserstoffen am Ausgang der Anlage [mol/h] |
| $n_{MSA, Reaktor, aus}$ | Stoffmengenstrom an Maleinsäureanhydrid am Reaktorausgang [mol/h] |
| $D_{MSA, Anlage, aus}$ | Stoffmengenstrom an Maleinsäureanhydrid am Ausgang der Anlage [mol/h] |

Beispiele

Bestimmung der mittleren Oxidationsstufe des Vanadiums

Die Bestimmung der mittleren Oxidationsstufe des Vanadiums erfolgte über potentiometrische Titration.

Zur Bestimmung werden jeweils 200 bis 300 mg der Probe unter Argonatmosphäre in eine Mischung aus 15 mL 50%-iger Schwefelsäure und 5 mL 85%-iger Phosphorsäure gegeben und unter Erwärmen gelöst. Die Lösung wird anschließend in ein Titrationsgefäß, welches mit zwei Pt-Elektroden ausgestattet ist, überführt. Die Titrationen werden jeweils bei 80°C durchgeführt. Zuerst erfolgt eine Titration mit 0,1 molarer Kaliumpermanganat-Lösung. Werden zwei Stufen in der potentiometrischen Kurve erhalten, so lag das Vanadium in einer mittleren Oxidationsstufe von· +3 bis kleiner +4 vor. Wird nur eine Stufe erhalten, so lag das Vanadium in einer Oxidationsstufe von +4 bis kleiner +5 vor.

Bei dem erstgenannten Fall (zwei Stufen / $+3 \leq V_{ox} < +4$) enthält die Lösung kein $V^{5+}$, das heißt das gesamte Vanadium wurde titrimetrisch erfaßt. Über den Verbrauch der 0,1 molaren Kaliumpermanganat-Lösung und der Lage der beiden Stufen wird die Menge an $V^{3+}$ und $V^{4+}$ berechnet. Der gewichtete Mittelwert ergibt dann die mittlere Oxidationsstufe.

Bei dem zweitgenannten Fall (eine Stufe / $+4 \leq V_{ox} < +5$) kann aus dem Verbrauch der 0,1 molaren Kaliumpermanganat-Lösung die Menge an $V^{4+}$ berechnet werden. Durch anschließende Reduktion des gesamten $V^{5+}$ der erhaltenen Lösung mit einer 0,1 molaren Ammoniumeisen(II)-sulfat-Lösung und erneute Oxidation mit 0,1 molarer Kaliumpermanganat-Lösung kann die Gesamtmenge an Vanadium berechnet werden. Die Differenz zwischen der Gesamtmenge an Vanadium und der Menge an $V^{4+}$ ergibt die ursprünglich vorhandene Menge an $V^{5+}$. Der gewichtete Mittelwert ergibt dann die mittlere Oxidationsstufe.

Bestimmung der Seitendruckfestigkeit der Hohlzylinder

Zur Bestimmung der Seitendruckfestigkeit wurden in nacheinander folgenden Messungen die Hohlzylinder mit der gerundeten Seitenfläche jeweils auf die plane Metall-Auflageplatte einer entsprechenden Meßeinrichtung gestellt. Die beiden planparallelen Dekkelflächen befanden sich somit in vertikaler Richtung. Nun wurde ein planer Metall-Stempel von oben mit einer Vorschubgeschwindigkeit von 1,6 mm/min auf den Hohlzylinder zugefahren und der zeitliche Verlauf der Krafteinwirkung auf den Hohlzylinder bis zu dessen Bruch aufgezeichnet. Die Seitendruckfestigkeit des einzelnen Hohlzylinders entspricht der maximal eingewirkten Kraft.

Zur Bestimmung der Seitendruckfestigkeit wurden unter Mittelwertbildung jeweils 30 Einzelmessungen durchgeführt.

Bestimmung der geometrischen Dichte

Die geometrische Dichte der Hohlzylinder ist definiert als der Quotient der Masse zum geometrischen Volumen der Hohlzylinder. Das geometrische Volumen ergibt sich aus den äußeren, makroskopischen Abmessungen der Hohlzylinder unter Berücksichtigung des äußeren Durchmessers, der Höhe und des Durchmessers des inneren Lochs.

Zur Bestimmung der geometrischen Dichte wurde eine statistisch belastbare Menge an Hohlzylindern vermessen, deren Masse bestimmt und die geometrischen Dichte rechnerisch ermittelt.

Bestimmung der mechanischen Stabilität durch Falltest

Zur Bestimmung der mechanischen Stabilität durch den Falltest wurden etwa 50 g geometrisch intakter und entstaubter Hohlzylinder durch ein 6,5 m langes Rohr mit einem Innendurchmesser von 21 mm nacheinander fallen gelassen und am Ende des Rohres mit einer Porzellanschale aufgefangen. Die aufgefangene Menge wurde anschließend per Hand sortiert und der Massenanteil der beschädigten Hohlzylinder (Hohlzylinder mit Rissen oder abgeplatzten Kanten, zertrümmerte Hohlzylinder, Bruchstücke, Splitter) bestimmt. Der Ausschuß nach dem Falltest ergibt sich nun aus dem Quotienten der Masse der beschädigten Hohlzylinder zur ursprünglich eingewogenen Masse.

**EP 1 487 575 B2**

Bestimmung des Abriebs

Zur Bestimmung des Abriebs wurden etwa 50 g entstaubter Hohlzylinder in eine Plexiglastrommel mit einem Innendurchmesser von 290 mm, einer Trommelhöhe von 40 mm und einem zwischen Drehachse und Außenwandung befindlichen, mit der Plexiglastrommel fest verbundenen, kreisförmig gekrümmten (Radius 80 mm) Plexiglaseinbau, welcher die gesamte Trommelhöhe von 40 mm umfasst, gegeben. Nun wurde die Plexiglastrommel, deren Drehachse sich in horizontaler Richtung befand, 18 Minuten lang mit 25 Umdrehungen pro Minute gedreht. Anschließend wurde der Abrieb der Probe abgesiebt, die verbliebenen Partikel entstaubt und zurückgewogen. Der Abriebswert ergibts sich nun aus dem Quotienten der Verlustmasse zur ursprünglich eingewogenen Masse.

Versuchsanlage

Die Versuchsanlage war ausgestattet mit einer Zufuhr-Einheit und einem Reaktorrohr. Der Ersatz eines Rohrbündelreaktors durch ein Reaktorrohr ist im Labor- oder Technikumsmaßstab sehr gut möglich, sofern die Abmessungen des Reaktorrohres im Bereich eines technischen Reaktorrohres liegen. Die Anlage wurde im "geraden Durchgang" betrieben.

Der Kohlenwasserstoff wurde mengengeregelt in flüssiger Form über eine Pumpe zugegeben. Als Sauerstoffhaltiges Gas wurde Luft mengengeregelt zugegeben. Triethylphosphat (TEP) wurde ebenfalls mengengeregelt, gelöst in Wasser, in flüssiger Form zugegeben.

Die RohrbündAlreaktor-Einheit bestand aus einem Rohrbündelreaktor mit einem Reaktorrohr. Die Länge des Reaktorrohrs betrug 6,5 m, der Innendurchmesser'22,3 mm. Innerhalb des Reaktorrohres befand sich in einem Schutzrohr mit 6 mm Außendurchmesser ein Multi-Thermoelement mit 20 Temperaturmeßstellen. Das Reaktorrohr war von einem temperierbaren Wärmeträger-Kreislauf umgeben und wurde von dem Reaktionsgasgemisch von oben nach unten durchströmt. Die oberen 0,3 m des Reaktorrohres waren mit Inertmaterial gefüllt und bildeten die Vorheizzone. Die Reaktionszone enthielt jeweils 2,2 L Katalysator. Als Wärmeträgermedium wurde eine Salzschmelze eingesetzt.

Direkt nach der Rohrbündelreaktor-Einheit wurde gasförmiges Produkt entnommen und der gaschromatographischen on-line Analytik zugeführt. Der Hauptstrom des gasförmigen Reaktoraustrags wurde aus der Anlage ausgeschleust.

Die Anlage wurde wie folgt betrieben:

| | |
|---|---|
| Konzentration an n-Butan am Reaktoreingang | = 2,0 Vol.-% |
| GHSV | = 2000 Nl/l$_{Katalysator}$ · h |
| Druck am Reaktorausgang | = 0,2 MPa abs |
| Konzentration an Triethylphosphat (TEP) | = 2 Volumen-ppm |
| Konzentration an Wasserdampf | = 1,5 Vol.-% |

Herstellung des Katalysatorprecursors

In einem mit Stickstoff inertisierten, über Druckwasser außenbeheizbaren 8 m³-Stahl/Email-Rührkessel mit Strombrechern wurden 6,1 m³ Isobutanol vorgelegt. Nach Inbetriebnahme des dreistufigen Impellerrührers wurde das Isobutanol unter Rückfluß auf 90°C aufgeheizt. Bei dieser Temperatur wurde nun über die Förderschnecke mit der Zugabe von 736 kg Vanadiumpentoxid begonnen. Nachdem nach ca. 20 Minuten etwa 2/3 der gewünschten Menge an Vanadiumpentoxid zugegeben wurden, wurde bei weiterer Zugabe an Vanadiumpentoxid mit der Einpumpung von 900 kg 105%-iger Phosphorsäure begonnen. Zur Reinigung der Pumpe wurden weitere 0,2 m³ Isobutanol nachgepumpt. Anschließend wurde das Reaktionsgemisch unter Rückfluß auf etwa 100 bis 108°C erhitzt und unter diesen Bedingungen 14 Stunden belassen. Im Anschluß daran wurde die heiße Suspension in eine zuvor mit Stickstoff inertisierte und beheizte Druckfilternutsche abgelassen und bei einer Temperatur von etwa 100°C bei einem Druck oberhalb der Filternutsche von bis zu 0,35 MPa abs abfiltriert. Der Nutschkuchen wurde durch stetiges Einleiten von Stickstoff bei 100°C und unter Rühren mit einem mittig angeordneten, in der Höhe verstellbaren Rührer innerhalb von etwa einer Stunde trokkengeblasen. Nach dem Trockenblasen wurde auf ca. 155°C aufgeheizt und auf einen Druck von 15 kPa abs (150 mbar abs) evakuiert. Die Trocknung wurde bis zu einem Rest-Isobutanolgehalt von < 2 Gew.-% im getrockneten Katalysator-Precursor durchgeführt.

Anschließend wurde das getrocknete Pulver 2 Stunden unter Luft in einem Drehrohr mit einer Länge von 6,5 m, einem Innendurchmesser von 0,9 m und innenliegenden spiralförmigen Wendeln behandelt. Die Drehzahl des Drehrohres betrug 0,4 U/min. Das Pulver wurde in einer Menge von 60 kg/h in das Drehrohr gefördert. Die Luftzufuhr betrug 100 m³/h. Die direkt an der Außenseite des Drehrohrs gemessenen Temperaturen der fünf gleichlangen Heizzonen

13

betrugen 250°C, 300°C, 340°C, 340°C und 340°C. Nach dem Abkühlen auf Raumtemperatur wurde der VPO-Precursor mit 1 Gew.-% Graphit innig vermischt und in einem Walzen-Kompaktor kompaktiert. Das Feingut im Kompaktat mit einer Partikelgröße < 400 μm wurde abgesiebt und erneut der Kompaktierung zugeführt. Das Grobgut mit einer Partikelgröße ≥ 400 μm wurde mit weiteren 2 Gew.-% Graphit vermischt und in einer Tablettiermaschine zu 5x3x2,5 mm Hohlzylindern (äußerer Durchmesser x Höhe x Durchmesser des inneren Lochs) mit einer Seitendruckfestigkeit von 11 N tablettiert. Um die erforderliche Menge an Katalysatorprecursor zu erhalten, wurden mehrere Ansätze durchgeführt.

Beispiel 1:

Kontinuierliche Kalzinierung des Katalysatorprecursors (erfindungsgemäß)

Etwa 2,7 t der erhaltenen 5x3x2,5 mm Hohlzylindern (siehe "Herstellung des Katalysatorprecursors") wurden in einer Schütthöhe von 9 bis 10 cm kontinuierlich auf das gasdurchlässige Förderband einer Bandkalziniervorrichtung aus zwei hintereinandergeschalteten, identischen Bandkalzinierapparaten mit insgesamt acht Kalzinierzonen gegeben. Die ersten 1,4 t wurden zur einmaligen Einstellung der Betriebsparameter der Bandkalziniervorrichtung verwendet. Da sie kein einheitliches Material darstellten, wurden sie im Folgenden nicht weiter betrachtet.

Die Bandkalziniervorrichtung wurde bei Atmosphärendruck betrieben. Zwischen den Kalzinierzonen 4 und 5 befand sich eine umkapselte Übergangszone. Jede der acht Kalzinierzonen umfasste zur Erzeugung einer Gaszirkulation jeweils einen Ventilator. Jede der acht Kalzinierzonen wurde mit der gewünschten Menge an gewünschtem Frischgas versorgt. Zur Erhaltung des gewünschten Atmosphärendrucks wurde eine entsprechende Gasmenge abgeführt. Das Volumen des pro Zeiteinheit in jeder Kalzinierungszone zirkulierenden Gases war größer als das Volumen des pro Zeiteinheit zu- oder abgeführten Gases. Zwischen zwei aufeinanderfolgenden Kalzinierungszonen befand sich zur Verringerung des Gasaustausches jeweils eine Trennwand, welche im Bereich des Stromes des Katalysatorprecursors offen war. Die Länge jeder Kalzinierzone $l_n$ betrug 1,45 m. Die Geschwindigkeit des Förderbandes wurde entsprechend der gewünschten Verweilzeit von etwa 2 Stunden pro Kalzinierzone eingestellt. Die einzelnen Zonen wurden wie in Tabelle 2 dargestellt betrieben:

Tabelle 2:

| Parameter zum Betrieb der Bandkalziniervorrichtung. | | |
|---|---|---|
| Zone | Temperatur | zugeführtes Frischgas |
| Kalzinierzone 1 | Aufheizen auf 250°C | Luft |
| Kalzinierzone 2 | Halten bei 250°C | Luft |
| Kalzinierzone 3 | Halten bei 250°C | Luft |
| Kalzinierzone 4 | Aufheizen auf 310°C | Luft |
| Übergangszone | Abkühlen auf 200°C | Luft |
| Kalzinierzone 5 | Aufheizen auf 425°C | $N_2$ |
| Kalzinierzone 6 | Halten bei 425°C | $N_2/H_2O$-Dampf (1:1) |
| Kalzinierzone 7 | Halten bei 425°C | $N_2/H_2O$-Dampf (1:1) |
| Kalzinierzone 8 | Abkühlen auf Raumtemperatur | $N_2$ |

Innerhalb aller acht Kalzinierzonen betrug die zeitliche Schwankung der Gastemperatur an jeder Stelle im Bereich des Stromes des Katalysatorprecursors nach der halben Länge der Kalzinierungszone $l_n/2$ jeweils ≤ 1,2°C und die lokale Differenz der Gastemperatur zwischen beliebigen Stellen im Bereich des Stromes des Katalysatorprecursors nach der halben Länge der Kalzinierungszone $l_n/2$ jeweils ≤ 1,5°C. Auf diese Weise wurden ca. 1,3 t fertiger Katalysator kontinuierlich hergestellt.

Während der Herstellung wurden in gegebenen Abständen Proben des fertigen Katalysators entnommen und hinsichtlich der mittleren Oxidationsstufe des Vanadiums, der Seitendruckfestigkeit und der geometrischen Dichte analysiert. Die erhaltenen Ergebnisse sind in Tabelle 3 wiedergegeben.

Wie den Ergebnissen zu entnehmen ist, kann durch das erfindungsgemäße Verfahren die für die Einstellung der Aktivität und Selektivität des Katalysators wichtige Größe der mittleren Oxidationsstufe des Vanadiums sicher handhabbar in einem sehr engen Bereich gehalten werden. Bei einem Mittelwert von 4,16 betrug der niedrigste gemessene Wert 4,14 und der höchste gemessene Wert 4,17.

Auch die Seitendruckfestigkeit und die geometrische Dichte konnte in engen Grenzen von 8,4 bis 11,4 N bei einem Mittelwert von 10,1 N sowie von 1,51 bis 1,58 g/mL bei einem Mittelwert von 1,55 g/mL gehalten werden. Der geringe Ausschuß nach dem Falltest und der gemessene Abrieb bestätigen die enge Streubreite ebenfalls.

Beispiel 2:

Katalytischer Test des Katalysators aus Beispiel 1

Mit einer repräsentativ ausgewählten Einzelprobe von 2,2 L des erfindungsgemäß hergestellten Katalysators aus Beispiel 1 wurde in der ober beschriebenen Versuchsanlage ein katalytischer Performancetest durchgeführt. Die Salzbadtemperatur wurde dabei derart eingestellt, dass ein n-Butan-Umsatz von etwa 85% resultierte. Die erhaltenen Ergebnisse sind in Tabelle 5 wiedergegeben.

Beispiel 3:

Diskontinuierliche Kalzinierung des Katalysatorprecursors (Vergleichsbeispiel)

Etwa 0,4 t der erhaltenen 5x3x2,5 mm Hohlzylindern (siehe "Herstellung des Katalysatorprecursors") wurden in einer Schütthöhe von 4 bis 5 cm auf gasdurchlässigen Blechen der Abmessung 0,5 × 1 m in einen Hordenofen gegeben. Der Hordenofen umfasste dabei insgesamt zehn Bleche in zwei nebeneinander befindlichen Reihen und war mit einer externen Gaszirkulation ausgestattet. Das zuvor entnommene und mit der gewünschten Menge an Frischgas angereicherte Gas wurde seitlich in den Hordenofen geleitet. Der kontinuierliche Gasabzug erfolgte mittig auf der Oberseite des Hordenofens. Dem entnommenen Gas wurde kontinuierlich die gewünschte Menge an Frischgas zugeführt, über einen Wärmetauscher und ein Gebläse geleitet und wie zuvor beschrieben dem Hordenofen wieder seitlich zugeführt. Der Hordenofen wurde bei einem geringen Überdruck von 3 kPa (30 mbar) betrieben. Zur Erhaltung des gewünschten Drucks wurde eine entsprechende Gasmenge nach dem Gebläse kontinuierlich abgeführt.

Zur Kalzinierung des Katalysatorprecursors wurde dieser im Hordenofen in einer Gasatmosphäre aus 2,5 Vol.-% Sauerstoff in Stickstoff mit einer Temperaturrampe von 2°C/min auf 350°C erhitzt und 25 Minuten unter diesen Bedingungen belassen. Es wurde stets frisches Gasgemisch zugeführt. Anschließend wurde die Gasatmosphäre durch eine Stickstoff-Wassersdampf-Atmosphäre (1:1) ausgetauscht und der Katalysatorprecursor mit 4°C/min auf 450°C aufgeheizt und 4 Stunden belassen. Zuletzt wurde unter einer Stickstoff-Atmosphäre abgekühlt.

Die im Vergleichsbeispiel (Beispiel 2) eingestellten Kalzinierungsbedingungen wurden in Vorversuchen im Hinblick auf die Herstellung eines Katalysators mit gegenüber dem Katalysator nach dem erfindungsgemäßen Beispiel 1 möglichst identischer mittlerer Oxidationsstufe des Vanadiums optimiert. Die Unterschiede in den einzelnen Parametern sind somit durch die erforderliche Optimierung begründet.

Nach der Kalzinierung wurden von beliebig ausgesuchten Stellen der Bleche Proben entnommen und hinsichtlich der mittleren Oxidationsstufe des Vanadiums, der Seitendruckfestigkeit und der geometrischen Dichte analysiert. Die erhaltenen Ergebnisse sind in Tabelle 4 aufgeführt.

Tabelle 4:

| Charakterisierung des im Hordenofen kalzinierten Katalysators. | | | |
|---|---|---|---|
| Katalysatorprobe (Nummer) | $V_{ox.}$ | SDF [N] | geometrische Dichte $d_p$ [g/mL] |
| 1 | 4,05 | 9 | 1,53 |
| 2 | 4,20 | 14 | 1,68 |
| 3 | 4,05 | 9 | 1,57 |
| 4 | 4,20 | 12 | 1,68 |
| Mittelwert: | 4,13 | 11 | 1,62 |
| $V_{ox.}$ = mittlere Oxidationsstufe des Vanadiums SDF = Seitendruckfestigkeit | | | |

Gegenüber den Charakterisierungsdaten des erfindungsgemäß durch kontinuierliche Kalzinierung in einer Bandkalziniervorrichtung kalzinierten Katalysators zeigt der diskontinuierlich im Hordenofen kalzinierte Katalysator sehr große Streubreiten. So variiert die mittlere Oxidationsstufe des Vanadiums der statistisch ausgewählten Proben von 4,05 bis 4,20, wobei der rechnerisch ermittelte Mittelwert bei 4,13 liegt. Auch die Seitendruckfestigkeit und die geometrische Dichte zeigen eine breite Streuung von 9 bis 14 N bei einem Mittelwert von 11 N beziehungsweise von 1,53 bis 1,68 g/mL bei einem Mittelwert von 1,62 g/mL.

Beispiel 4:

Katalytischer Test des Katalysators aus Beispiel 3

Mit 2,2 L einer statistischen Mischung des diskontinuierlich im Hordenofen kalzinierten Katalysators aus Beispiel 3 wurde in der ober beschriebenen Versuchsanlage ein katalytischer Performancetest durchgeführt. Die Salzbadtemperatur wurde dabei derart eingestellt, dass ein n-Butan-Umsatz von etwa 85% resultierte. Die erhaltenen Ergebnisse sind in Tabelle 5 wiedergegeben.

Tabelle 5:

| Ergebisse der katalytischen Tests | | |
|---|---|---|
| | Katalysator nach Beispiel 1 (Kontinuierliche Kalzination im Bandkalzinierer) | Katalysator nach Beispiel 3 (Diskontinuierliche Kalzination im Hordenofen) |
| Salzbadtemperatur [°C] | 395 | 410 |
| Temperatur des Hot Spots [°C] | 425 | 434 |
| Druckverlust [MPa] | 0,13 | 0,13 |
| Umsatz U [%] | 84,8 | 85,0 |
| Ausbeute A [%] | 57,5 | 56,7 |

Ein Vergleich der Ergebnisse mit denen des erfindungsgemäß hergestellten Katalysators von Beispiel 1 zeigt, dass aufgrund der, großen Streubreite des Vergleichskatalysators eine um 15°C höhere Salzbadtemperatur (410°C gegenüber 395°C) zur Einstellung der 85% n-Butan-Umsatz erforderlich ist. Als Folge davon wird beim Vergleichskatalysator eine um 9°C höhere Hot-Spot-Temperatur erreicht. Das niedrigere Temperaturniveau, welches beim erfindungsgemäßen Katalysator erreicht wird, ist aus vielen Gründen vorteilhaft. So wird beispielsweise der Katalysator thermisch geringer belastet, was zu einer höheren Lebensdauer führt. Die Gefahr des "Durchgehens" der Reaktion ist ebenfalls aufgrund des niedrigeren Temperaturniveaus vermindert. Des weiteren arbeitet der Katalysator selektiver, was bei gleichem Umsatz zu einer höheren Ausbeute an Wertprodukt führt. Dies ist auch aus den experimentellen Daten ersichtlich (57,5% Ausbeute beim erfindungsgemäß hergestellten Katalysator gegenüber 56,7% Ausbeute im Vergleichsbeispiel).

Tabelle 3:

| Charakterisierung des erfindungsgemäß hergestellten Katalysators. | | | | | |
|---|---|---|---|---|---|
| Katalysatorprobe nach x t | $V_{ox.}$ | SDF [N] | geometrische Dichte $d_p$ [g/mL] | Ausschuß nach Fall-test[Gew.-%] | Abrieb [Gew.-%] |
| 1,47 | 4,16 | 8,7 | 1,51 | 2,0 | 0,8 |
| 1,70 | 4,16 | 10,1 | 1,55 | 3,6 | 0,4 |
| 1,99 | 4,16 | 9,9 | 1,55 | 4,7 | 0,9 |
| 2,19 | 4,14 | 11,2 | 1,55 | 3,5 | 0,7 |
| 2,35 | 4,15 | 8,4 | 1,56 | 3,3 | 0,7 |
| 2,51 | 4,17 | 11,4 | 1,58 | 2,1 | 0,9 |
| 2,67 | 4,17 | 11,3 | 1,58 | 4,0 | n.b. |

(fortgesetzt)

| Charakterisierung des erfindungsgemäß hergestellten Katalysators. | | | | | |
|---|---|---|---|---|---|
| Katalysatorprobe nach x t | $V_{ox.}$ | SDF [N] | geometrische Dichte $d_p$ [g/mL] | Ausschuß nach Fall-test[Gew.-%] | Abrieb [Gew.-%] |
| Mittelwert: | 4,16 | 10,1 | 1,55 | 3,3 | 0,7 |
| n.b. = nicht bestimmt<br>$V_{ox}$ = mittlere Oxidationsstufe des Vanadiums<br>SDF = Seitendruckfestigkeit | | | | | |

**Patentansprüche**

1. Verfahren zur Herstellung eines für die heterogenkatalytische Gasphasenoxidation eines Kohlenwasserstoffs mit mindestens vier Kohlenstoffatomen zu Maleinsäureanhydrid geeigneten, Vanadium, Phosphor und Sauerstoff enthaltenden Katalysators, bei dem man einen entsprechenden Vanadium, Phosphor und Sauerstoff enthaltenden Katalysatorprecursor, welcher Partikel mit einem gemittelten Durchmesser von mindestens 2 mm aufweist, durch Kalzinierung in eine katalytisch aktive Form überführt, **daduch gekennzeichnet**, dass man einen Strom des Katalysatorprecursors zur Kalzinierung mit im Wesentlichen konstanter Geschwindigkeit auf. einem Förderband durch mindestens eine Kalzinierungszone n der Länge $l_n$ führt, wobei die zeitliche Schwankung der Gastemperatur vom Sollwert an jeder Stelle im Bereich des Stromes des Katalysatorprecursors nach der halben Länge der Kalzinierungszone $l_n/2$ jeweils $\leq 5°C$ und die lokale Differenz der Gastemperatur zwischen beliebigen Stellen im Bereich des Stromes des Katalysatorprecursors nach der halben Länge der Kalzinierungszone $l_n/2$ jeweils $\leq 5°C$ betragen, und **dadurch gekennzeichnet, dass** man den Katalysatorprecursor

   (a) in mindestens einer Kalzinierungszone in einer oxidierenden Atmosphäre mit einem Sauerstoff-Gehalt von 2 bis 21 Vol.-% auf eine Temperatur von 200 bis 350°C aufheizt und unter diesen Bedingungen bis zur Einstellung der gewünschten mittleren Oxidationsstufe des Vanadiums beläßt; und
   (b) in mindestens einer weiteren Kalzinierungszone in einer nicht-oxidierenden Atmosphäre mit einem Sauerstoff-Gehalt von $\leq 0,5$ Vol. -% und einem Wasserstoffoxid-Gehalt von 20 bis 75 Vol.-% auf eine Temperatur von 300 bis 50.0°C aufheizt und $\geq 0,5$ Stunden unter diesen Bedingungen beläßt.

2. Verfahren nach Anspruch 1, **dadurch gekennzeichnet, dass** man ein gasdurchlässiges Förderband einzetzt und in der Kalzinierungszone n senkrecht zur Fortbewegungsrichtung des Katalysatorprecursors einen Gasstrom durch den Strom des Katalysatorprecursors leitet.

3. Verfahren nach den Ansprüchen 1 bis 2, **dadurch gekennzeichnet, dass** man in der Kalzinierungszone n eine Gaszirkulation einstellt, bei der das Volumen des pro Zeiteinheit in der Kalzinierungszone n zirkulierenden Gases größer ist als das Volumen des pro Zeiteinheit in der Kalzinierungszone n zugeführten Gases.

4. Verfahren nach Anspruch 3,' **dadurch gekennzeichnet, dass** man zum Erzeugen der Gaszirkulation einen Ventilator einsetzt.

5. Verfahren nach den Ansprüchen 1 bis 4, **dadurch gekennzeichnet, dass** die zeitliche Schwankung der Gastemperatur vom Sollwert an jeder Stelle im Bereich des Stromes des Katalysatorprecursors nach der halben Länge der Kalzinierungszone $l_n/2$ jeweils $\leq 2°C$ beträgt.

6. Verfahren nach den Ansprüchen 1 bis 5, **dadurch gekennzeichnet, dass** die lokale Differenz der Gastemperatur zwischen beliebigen Stellen im Bereich des Stromes des Katalysatorprecursors nach der halben Länge der Kalzinierungszone $l_n/2 \leq 3°C$ beträgt.

7. Verfahren nach den Ansprüchen 1 bis 6, **dadurch gekennzeichnet, dass** man den Strom des Katalysatorprecursors zur Kalzinierung durch mindestens zwei Kalzinierungszonen führt und die Temperaturen der einzelnen Kalzinierungszonen voneinander unabhängig regelbar sind.

8. Verfahren nach den Ansprüchen 1 bis 7, **dadurch gekennzeichnet, dass** man einen Katalysatorprecursor einsetzt, welcher im Wesentlichen eine hohlzylinderförmige Struktur aufweist.

9. Verfahren nach den Ansprüchen 1 bis 8, **dadurch gekennzeichnet, dass** man einen Katalysator herstellt, welcher ein Phosphor/Vanadium-Atomverhältnis von 0,9 bis 1,5, eine mittlere Oxidationsstufe des Vanadiums von +3,9 bis +4,4, eine BET-Oberfläche von 10 bis 50 $m^2$/g, ein Porenvolumen von 0,1 bis 0,5 ml/g und eine Schüttdichte von 0,5 bis 1,5 kg/l aufweist.

**Claims**

1. A process for preparing a vanadium, phosphorus, and oxygen catalyst suitable for heterogeneously catalyzed gas-phase oxidation of a hydrocarbon comprising at least four carbon atoms to maleic anhydride, in which a corresponding vanadium, phosphorus, and oxygen catalyst precursor containing particles having an averaged diameter of at least 2 mm is converted into a catalytically active form by calcining, which comprises passing a stream of the catalyst precursor for calcining at substantially constant speed through at least one calcining zone n of length $l_n$ on a conveyor belt, the fluctuation in the gas temperature from the setpoint value over time at each point in the region of the stream of the catalyst precursor after half the length of the calcining zone, $l_n/2$, being in each case $\leq 5°C$ and the local difference in gas temperature between any two points in the region of the stream of the catalyst precursor after half the length of the calcining zone, $l_n/2$, being in each case $\leq 5°C$, and wherein the catalyst precursor

   (a) is heated to a temperature of from 200 to 350°C in at least one calcining zone in an oxidizing atmosphere having an oxygen content of from 2 to 21% by volume and is left under these conditions until the desired average oxidation state of the vanadium is reached; and
   (b) is heated to a temperature of from 300 to 500°C in at least one further calcining zone in a nonoxidizing atmosphere having an oxygen content of $\leq 0.5\%$ by volume and a hydrogen oxide content of from 20 to 75% by volume and is left under these conditions for $\geq 0.5$ hour.

2. The process according to claim 1, wherein a gas-permeable conveyor belt is used and a gas stream is passed through the stream of the catalyst precursor perpendicularly to its direction of advance in the calcining zone n.

3. The process according to either of claims 1 and 2, wherein a gas circulation system is set up in the calcining zone n, the volume of gas circulating in the calcining zone n per unit time being greater than the volume of gas supplied to the calcining zone n per unit time.

4. The process according to claim 3, wherein a fan is used to generate the gas circulation system.

5. The process according to any of claims 1 to 4, wherein the fluctuation in the gas temperature from the setpoint value over time at each point in the region of the stream of the catalyst precursor after half the length of the calcining zone, $l_n/2$, is in each case $\leq 2°C$.

6. The process according to any of claims 1 to 5, wherein the local difference in gas temperature between any two points in the region of the stream of the catalyst precursor after half the length of the calcining zone, $l_n/2$, is $\leq 3°C$.

7. The process according to any of claims 1 to 6, wherein the stream of the catalyst precursor for calcining is passed through at least two calcining zones and the temperatures of the individual calcining zones can be regulated independently of one another.

8. The process according to any of claims 1 to 7, wherein a catalyst precursor is used which has substantially a hollow cylindrical structure.

9. The process according to any of claims 1 to 8, wherein a catalyst is prepared which has a phosphorus/vanadium atomic ratio of from 0.9 to 1.5, an average oxidation state of the vanadium of from +3.9 to +4.4, a BET surface area of from 10 to 50 $m^2$/g, a pore volume of from 0.1 to 0.5 ml/g, and a bulk density of from 0.5 to 1.5 kg/l.

**Revendications**

1. Procédé de préparation d'un catalyseur contenant du vanadium, du phosphore et de l'oxygène, qui est approprié pour l'oxydation en phase gazeuse à catalyse hétérogène d'un hydrocarbure comportant au moins quatre atomes de carbone en anhydride maléique, dans lequel on convertit en une forme catalytiquement active un précurseur de catalyseur correspondant contenant du vanadium, du phosphore et de l'oxygène et présentant des particules ayant un diamètre moyen d'au moins 2 mm, par calcination, **caractérisé en ce qu'**on guide un courant du précurseur de catalyseur vers la calcination à une vitesse sensiblement constante, sur une bande transporteuse et au travers d'au moins une zone de calcination n de la longueur $l_n$, la variation dans le temps de la température gazeuse depuis la valeur de consigne à chaque endroits dans la zone du courant du précurseur de catalyseur après la demi-longueur de la zone de calcination $l_n/2$ étant respectivement $\leq 5°C$, et la différence locale de la température gazeuse entre des endroits quelconques dans la zone du courant du précurseur de catalyseur après la demi-longueur de la zone de calcination $l_n/2$ étant respectivement $\leq 5°C$, et **caractérisé en ce que**,

(a) on chauffe le précurseur de catalyseur dans au moins une zone de calcination dans une atmosphère oxydante avec une teneur en oxygène de 2 à 21 % en volume à une température de 200 à 350°C et, dans ces conditions, on l'abandonne jusqu'à l'ajustement du degré d'oxydation moyen souhaité du vanadium, et

(b) on chauffe le précurseur de catalyseur dans au moins une autre zone de calcination dans une atmosphère non oxydante avec une teneur en oxygène de $\leq 0,5$ % en volume et une teneur en oxyde d'hydrogène de 20 à 75 % en volume à une température de 300 à 500°C et on l'abandonne dans ces conditions pendant $\geq 0,5$ heure.

2. Procédé suivant la revendication 1, **caractérisé en ce qu'**on met en oeuvre une bande transporteuse perméable aux gaz et **en ce que**, dans la zone de calcination n, on guide un courant gazeux à travers le courant du précurseur de catalyseur perpendiculairement à la direction d'avancement du précurseur de catalyseur.

3. Procédé suivant les revendications 1 et 2, **caractérisé en ce que**, dans la zone de calcination n, on ajuste une circulation gazeuse à laquelle le volume du gaz circulant par unité de temps dans la zone de calcination n est plus grand que le volume du gaz amené par unité de temps dans la zone de calcination n.

4. Procédé suivant la revendication 3, **caractérisé en ce que**, pour la production de la circulation du gaz, on met en oeuvre un ventilateur.

5. Procédé suivant les revendications 1 à 4, **caractérisé en ce que** la variation dans le temps de la température gazeuse depuis la valeur de consigne à chaque endroit dans la zone du courant du précurseur de catalyseur après la demi-longueur de la zone de calcination $l_n/2$ est respectivement $\leq 2°C$.

6. Procédé suivant les revendications 1 à 5, **caractérisé en ce que** la différence locale de la température gazeuse entre des endroits quelconques dans la zone du courant du précurseur de catalyseur après la demi-longueur de la zone de calcination $l_n/2$ est $\leq 3°C$.

7. Procédé suivant les revendications 1 à 6, **caractérisé en qu'**on guide le courant du précurseur de catalyseur vers la calcination au travers d'au moins deux zones de calcination et en ce que les températures des différentes zones de calcination sont réglables indépendamment l'une de l'autre.

8. Procédé suivant les revendications 1 à 7, **caractérisé en ce qu'**on met en oeuvre un précurseur de catalyseur qui présente sensiblement une structure en forme de cylindre creux.

9. Procédé suivant les revendications 1 à 8, **caractérisé en ce qu'**on prépare un catalyseur qui présente un rapport atomique phosphore/vanadium de 0,9 à 1,5, un degré d'oxydation moyen du vanadium de +3 , 9 à +4, 4, une surface spécifique BET de 10 à 50 m$^2$/g, un volume poreux de 0,1 à 0,5 ml/g et une densité apparente de 0,5 à 1,5 kg/litre.

Figur 1

Figur 2

**EP 1 487 575 B2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4567158 A
- US 4996179 A
- US 5137860 A
- US 5506187 A
- US 5530144 A
- US 5773382 A
- US 5275996 A
- US 5641722 A
- US 5095125 A

- US 4933312 A
- WO 9712674 A
- WO 9526817 A
- US 5296436 A
- US 5158923 A
- US 4795818 A
- EP 0593646 A
- US 5168090 A

**In der Beschreibung aufgeführte Nicht-Patentliteratur**

- MALEIC AND FUMARIC ACIDS, Maleic Anhydride - Production. Ullmann's Encyclopedia of Industrial Chemistry. 2000